(19) Europäisches Patentamt
European Patent Office
Office européen des brevets

(11) EP 3 882 279 A1

(12) EUROPEAN PATENT APPLICATION

(43) Date of publication:
**22.09.2021 Bulletin 2021/38**

(21) Application number: **20163463.1**

(22) Date of filing: **16.03.2020**

(51) Int Cl.:
***C08B 37/00*** *(2006.01)*
***A61K 31/711*** *(2006.01)*
***A61L 27/20*** *(2006.01)*
***C08J 3/24*** *(2006.01)*
***C08L 99/00*** *(2006.01)*
***A61K 8/73*** *(2006.01)*
***A61K 47/36*** *(2006.01)*
***C08H 99/00*** *(2010.01)*
***C08L 5/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Perpetuum CropScience BVBA**
**2610 Antwerpen (BE)**

(72) Inventors:
- **HOFENK, Jeroen**
  **2000 Antwerpen (BE)**
- **TROCH, James**
  **2610 Antwerpen (BE)**

(74) Representative: **Clerinx, Peter Albert I.**
**Clarity IP bvba**
**Riddersstraat 12**
**3890 Montenaken (BE)**

# (54) GENIPIN-CROSSLINKED PDRN-SACRAN BIOPOLYMER SCAFFOLDS

(57) A biopolymer is disclosed that comprises a polydeoxyribonucleotide (PDRN), or a derivative or modification thereof, having a molecular weight in the range of 400kDa to 3200 kDa, and an anionic and ampholytic heteropolysaccharide having sulfate and carboxyl groups and a molecular weight in the range of 14MDa and 16MDa, in which the polydeoxyribonucleotide and the heteropolysaccharide are crosslinked by genipin or an analogue or derivative thereof. Furthermore, the invention relates to methods of producing such biopolymer and uses thereof.

EP 3 882 279 A1

## Description

### Field of the invention

The present invention relates to the field of biopolymers, e.g. biodegradable biopolymers, and, more specifically, to a biopolymer and related compositions, uses thereof, and methods of preparation of such biopolymer.

### Background of the invention

Biodegradable biopolymers can naturally occur as polymeric biomolecules formed during the life cycle of green plants, animals, bacteria, and fungi. Such biomolecules may typically have an advantageously high degree of biocompatibility. Animal protein-based biopolymers include, or form a substantial component of, well-known products such as wool, silk, gelatin, and collagen. Other examples include polysaccharides, such as cellulose and starch, and carbohydrate polymers produced by bacteria and fungi. Biopolymers, and particularly those of carbohydrate origin, are promising for biomedical applications in various forms, e.g. due to their advantageously low toxicity, low antigenicity, high bio-activity, biodegradability, stability, processability into complicated shapes with appropriate porosity to support cell growth, mechanical properties, and renewable nature.

The United States Congress Office of Technology Assessment classifies such biopolymers into nucleic acids, proteins, polysaccharides, polyhydroxyalkanoates and polyphenols.

Biopolymers may be generated from renewable sources and may be easily biodegradable because of the oxygen and nitrogen atoms found in their structural backbone. Biodegradation converts them to $CO_2$, water, biomass, humid matter, and other, typically harmless, natural substances. Biopolymers can thus be naturally recycled by biological processes.

Bioactive properties of biopolymers, such as antimicrobial, immune-modulatory, cell proliferative and angiogenic properties, create a microenvironment that is favorable for the healing process and provide a plethora of applications in pharmaceutical, medical and cosmetic applications. Furthermore, one of the major advantages of biopolymers is their ability to absorb large volumes of water when in the dry state, while being able to donate water when hydrated.

The foundation of polymer science was laid with the discovery of cellulose in 1838 by the French chemist Anselme Payen, who isolated it from plant matter and determined its chemical formula, being identified as a covalently linked, high-molecular-weight macromolecule.

Cellulose is an abundant naturally occurring polymer of glucose, found as the main constituent of plants and natural fibers such as cotton and linen. Some bacteria (e.g., Acetobacter xylinum) can also synthesize cellulose. While plant cellulose is chemically identical to microbial or bacterial cellulose, it can possess a different macromolecular structure and physical properties. Natural cotton fibers and regenerated (artificial) cellulose fibers, such as viscose, modal, and lyocell, are used in many medical applications. In devices for controlled drug delivery, e.g. in the form of solid tablets, cellulose ether can be used, which allows a swelling-driven release of the drug when physiological fluids come into contact with the tablet. When the cellulose ether on the tablet surface swells, chain entanglements and a physical hydrogel are formed. As swelling proceeds from the surface to the glassy core of the tablet, the drug progressively dissolves in water and diffuses out from the polymer network.

Cellulose can also be used as scaffolds for regenerative medicine due to its excellent biocompatibility and good mechanical properties. For example, bacterial cellulose may be suitable for use in wound dressings or other wound healing applications, particularly in view of its purity and high water retention capacity.

Collagen is another great example of a biodegradable biopolymer. This is a predominant structural material in vertebrates, and the most abundant mammalian protein, e.g. accounting for about 20% to 30% of total body proteins. Collagen is synthesized by fibroblasts, which usually originate from pluripotential adventitial cells or reticulum cells. The elementary monomer molecule of collagen is rod-shaped, with a length and width of, respectively, about 3000 Å and 15 Å, and has an approximate molecular weight of 300 kDa. It is easily absorbable in the body and has a very low antigenicity. Moreover, it is non-toxic, biocompatible and biodegradable, and has a high tensile strength and high affinity with water.

In biomedical applications, collagen is used in numerous applications. For example, it can be used in tissue-based devices, such as prosthetic heart valves and vascular prostheses, which has contributed to an increased life expectancy of cardiac patients and lower risks of infection.

Collagen can also be used in drug delivery systems. However, its main application may be in collagen films used as barrier membrane. Collagen films, sheets and/or discs can be used in the treatment of tissue infections, such as infected corneal tissue or liver cancer. A soluble ophthalmic insert, in the form of a wafer or a film, is known in the art, as a drug delivery system for the treatment of infected corneal tissue using a high dose of antibiotic agents, such as gentamycin and tetracycline. Collagen films have also been used as a matrix for gene delivery carriers to promote bone formation.

Another useful medical application is the use of collagen sponges in the management of severe burns and as

a dressing for severe wounds. Collagen dressings are prepared in a variety of different forms including, sponges, membrane sheets and powder, which have advantageous biological properties for such applications. In tissue engineering, collagen-based implants are widely used as vehicles for delivery of cultured skin cells and as drug carriers for skin replacement and burn wounds treatment. They have also been used as bone substitute, due to its osteoinductive activity, and as implantable carriers for bone inducing proteins, such as bone morphogenetic protein 2 (rhBMP-2).

As a haemostat, collagen achieves rapid coagulation of blood through its interaction with the platelets and can provide a temporary framework while the host cells regenerate their own fibrous stroma. The use of collagen-based haemostats has been proposed for reducing blood loss in generalized bleeding in a wide range of tissues, and for managing wounds to cellular organs, such as the liver or spleen.

Another widely used and studied biodegradable biopolymer is chitosan. Chitosan is a derivative of the natural polysaccharide, chitin. Chitin was first isolated and characterized from mushrooms, by the French chemist Henri Braconnot in 1811. It is known as the second most abundant biopolymer, i.e. it is the most abundant polysaccharide in nature after celluloses. Chitin is the main component of the exoskeleton of crustaceans and insects, and also occurs in nematodes and in the cell wall of yeast and fungi. Chitosan has an excellent biocompatibility and other advantageous properties, such as good bioactivity, biodegradability, selective permeability, polyelectrolyte action, antimicrobial activity, ability to form gel and film, chelation ability and absorptive capacity. These peculiar properties enable a wide range of useful applications of chitosan, such as a drug carrier for controlled release, an anti-bacterial, and an anti-acid. It inhibits the bacterial plaque formation and decalcification of dental enamel, promotes osteogenesis, can act as a fat absorbent and promotes the healing of ulcers and lesions.

Chitosan has an in vivo stimulatory effect on nitric oxide production and modulates peroxide production. Neutrophil activation can be inhibited by administration of chitosans having a low molecular weight and oxidation of serum albumin is commonly observed in patients undergoing hemodialysis, resulting in reduction of oxidative stress associated with uremia.

In drug delivery, chitosan may potentially improve drug absorption and stabilization of drug components to increase drug targeting. In addition, chitosan can protect DNA and increase the expression period of genes. Chitin or chitosan derivatives, which were conjugated with some kinds of anticancer agents, can execute better anticancer effects with gradual release of free drug in the cancer tissues.

As an anti-inflammatory agent, chitosan can partially inhibit the secretion of both IL-8 and TNF-$\alpha$ from mast cells, demonstrating that water-soluble chitosan has the potential to reduce the allergic inflammatory response. Chitosan promotes phagocytosis and production of osteopontin and leukotriene B by polymorphonuclear leukocytes, production of interleukin-1, transforming growth factor b1 and platelet-derived growth factor by macrophages, and production of interleukin-8 by fibroblasts, enhancing immune responses.

As a composite for tissue engineering, chitosan and hydroxyapatite are one of the best bioactive biomaterials in bone tissue engineering. Different types of polysaccharides from marine sources are used for the management of bone diseases, such as osteoporosis and arthritis. As a functional wound dressing, a hydrogel sheet composed of a blended powder of alginate, chitin/chitosan and fucoidan is known for creating a moist environment for rapid wound healing. It is highly biocompatible and biodegradable, has a porous structure, and is suitable for cell ingrowth, osteoconduction and intrinsic antibacterial nature. Chitosan has been developed considerably in biomedical applications, e.g. cartilage tissue engineering, wound healing and orthopedic applications. Degradable polymeric implants have the benefit that the need for a second surgical operation for removal can be avoided. Chitosan/hydroxyapatite composite materials are promising to mimic both the organic as the inorganic portion of natural bone.

Chitosan as an antimicrobial agent shows antimicrobial action in a great variety of microorganisms, including algae, fungi and bacteria. It has shown antimicrobial activity against gram-positive bacteria and various species of yeast. A chitosan gel, derived from dilution in acetic acid, was suggested as a preventive and therapeutic material for dental caries.

Hyaluronic acid is also widely distributed in the extracellular matrix and the joint liquid of mammals and has been approved for injections by the Food and Drug Administration (FDA). It is a biocompatible and biodegradable mucoadhesive polysaccharide with negative charge. The first medical application of hyaluronan for humans was as a vitreous substitution/replacement during eye surgery in the late 1950s. Hyaluronic acid polymers are used in the preparation of gels for delivery of drugs to the eye and installation into other cavities. Along with other polymers like alginic acid, HPMC (Hydroxypropyl methyl cellulose), poloxamers etc. can be used for achieving the desired property in drug delivery systems.

Corneal shields based on hyaluronic acid have demonstrated prolonged steroid delivery, with a consummate smoothing of dosage profile. Other applications of hyaluronic acid include: (i) wound healing by extracellular regeneration; (ii) epithelial regeneration; (iii) topical treatment of dry eye syndrome and Sjögren's syndrome; (iv) as a viscosity agent in pulmonary pathology for achieving alveolar patency; (v) a commercial preparation available for intra-articular injection; (vi) As a filler in rejuvenative medicine for wrinkles and cutaneous lines.

Viscosupplementation with hyaluronic acid products helps to improve the physiological environment in an

osteoarthritic joint by supplementing the shock absorption and lubrication properties of osteoarthritic synovial fluid. The rationale for using viscosupplementation is to restore the protective viscoelasticity of synovial hyaluronan, decrease pain, and improve mobility. Esterified hyaluronic acid has also been used to prevent bacterial adhesion to dental implants, intraocular lenses, and catheters. Hyaluronic acid is an appropriate choice for a matrix to support dermal regeneration and augmentation. As a result of its ability to form hydrated, expanded matrices, Hyaluronic acid has also been successfully used in cosmetic applications such as soft tissue augmentation.

PDRN is another biodegradable biopolymer that possesses several activities: tissue repairing, anti-ischemic, and anti-inflammatory. These therapeutic properties may suggest a use in regenerative medicine and in diabetic foot ulcers. PDRN holds a mixture of deoxyribonucleotides with molecular weights ranging between 50 and 1500 KDa, it is derived from a controlled purification and sterilization process of Oncorhynchus mykiss (Salmon Trout), Oncorhynchus keta (Chum Salmon), or sturgeon sperm DNA. The procedure guarantees the absence of active protein and peptides that may cause immune reactions. In vitro and in vivo experiments have suggested that PDRN most relevant mechanism of action is the engagement of adenosine A2A receptors. Besides engaging the A2A receptor, PDRN offers nucleosides and nucleotides for the so called "salvage pathway." The binding to adenosine A2A receptors is a unique property of PDRN and seems to be linked to DNA origin, molecular weight and manufacturing process. In this context, PDRN represents a new advancement in pharmacotherapy. In fact, adenosine and dipyridamole are non-selective activators of adenosine receptors and they may cause unwanted side effects; while regadenoson, the only other A2A receptor agonist available, has been approved by the FDA as a pharmacological stress agent in myocardial perfusion imaging.

PDRN has also been successfully used in cosmetic applications as a so-called injectable or topical "skin-healer".

Biodegradable polymers such as chitosan and hyaluronic acid need to be crosslinked in order to modulate their general properties and to last long enough for delivering drugs over a desired period of time. Various chemicals have been used for crosslinking chitosan, such as glutaraldehyde, tripolyphosphate, ethylene glycol, diglycidyl ether and diisocyanate. However, the synthetic crosslinking reagents are all more or less cytotoxic and may impair the biocompatibility of a chitosan, hyaluronic acid or other biodegradable polymer delivery system. Hence, efforts were made to provide crosslinking reagents that have low cytotoxicity and that form stable and biocompatible crosslinked products.

A need exists in the art for novel biodegradable biopolymers that have a wide pleiotropic anti-inflammatory action, and good properties with respect to toxicity, antigenicity, bio-activity, biodegradability, stability, processability, mechanical properties and renewable nature. There is also a clinical need for biocompatible drug carriers that release an effective quantity of drug over a period of time for prolonged therapeutic effects. Drugs are usually admixed or entrapped physically within a polymer framework for slow drug release. Plastic polymers that are suitable as a drug carrier may, however, lack sufficient biocompatibility, or biocompatible plastic polymers may be ineffective in containing a specific drug and/or releasing the drug in an timely manner for effective therapies. Therefore, a need remains for producing biodegradable biopolymers with the beforementioned capabilities, preferably by a low-cost and efficient process and/or in a highly purified state.

## Summary of the invention

It is an object of embodiments of the present invention to provide in a biopolymer, methods of producing such biopolymer and uses of such biopolymer. The biopolymer may have anti-inflammatory uses, such that such biopolymer can be used in compositions for applications in oral hygiene products, wound-care, cosmetics, pharmaceuticals, food, and/or as mucoadhesive carrier of APIs. The novel biopolymers of the present invention may be PDRN-genipin-Sacran scaffolds, PDRN-Genipin scaffolds, or Sacran-Genipin Scaffolds, or analogues thereof, wherein genipin, or an analogue or derivative thereof, functions as a natural crosslinker.

It is an advantage of embodiments of the present invention that a useful compound is provided for treatment of inflammatory gum diseases and/or as a wound healing agent.

It is an advantage of embodiments of the present invention that a biopolymer is provided that has low toxicity, low antigenicity, high bio-activity, good biodegradability, good stability, good processability into complicated shapes, e.g. with appropriate porosity to support cell growth, and/or good mechanical properties.

It is an advantage of embodiments of the present invention that genipin, analogues or derivatives thereof has good crosslinking properties.

It is an advantage of embodiments of the present invention that the provided biopolymer is renewable in nature, e.g. can be generated from renewable sources and are easily biodegradable, e.g. into harmless and/or reusable products.

In a first aspect, the present invention relates to a biopolymer comprising a polydeoxyribonucleotide (PDRN) and an anionic and ampholytic heteropolysaccharide having sulfate and carboxyl groups. The polydeoxyribonucleotide has a molecular weight in the range of 400 000 Dalton (or 400 kDa) to 3 200 000 Dalton (or 3.2 MDa), e.g. in the range of 2.0 MDa to 2.8 MDa, e.g. 2.4 MDa. The heteropolysaccharide has a molecular weight in the range of 14 000 000 Dalton (or 14 MDa) to 16 000 000 Dalton (or 16 MDa). The polydeoxyribonucleotide and the heteropolysaccharide are crosslinked by genipin, a water soluble aglycon, or an analogue or derivative thereof.

PDRN may advantageously have pharmacological activity without transmitting genetic information, e.g. unlike DNA and/or RNA fragments. PDRN may refer to pure PDRN, or an active form of 95% or higher purity. PDRN may be obtained from sturgeon, salmon (Oncorhynchus keta), or trout (Oncorhynchus mykiss) semen, e.g. by subjecting the material to a high heat treatment. The INCI name is Sodium DNA. For example, PDRN may be a sodium DNA extracted from semen or testis tissue. PDRN may be derived from any one or more of fish, animals, and plants. Specifically, the PDRN may be derived from sperm, semen and/or testes of, e.g. preferably, fish. PDRN may be composed of a DNA polymer, which may promote DNA synthesis and rapid tissue regeneration. For example, PDRN may be anti-inflammatory and may shorten wound healing time and/or activate cell regeneration. PDRN may activate the production of proteins and promote the differentiation of cells. The G-C content (%) of PDRN may be about 40%. PDRN may also comprise a mixture of DNA and/or RNA fragments. PDRN in the context of the present disclosure may also refer to derivatives or analogues of PDRN.

A biopolymer in accordance with embodiments of the present invention may be a biopolymer as obtainable by a method in accordance with embodiments of the present invention.

In a polymer in accordance with embodiments of the present invention, the PDRN may be derived from the testes or milt of Onchorhynchus species.

In a biopolymer in accordance with embodiments of the present invention, the heteropolysaccharide may be sacran.

Sacran may refer to a sulfated polysaccharide, e.g. in accordance with the CAS Registry Number 1039552-36-7. Sacran may have a repeat structure of a sugar chain unit where a sugar constituent having a hexose structure and a sugar constituent having a pentose structure are conjugated together in a linear chain or a branched chain through an $\alpha$-glycoside bond or a $\beta$-glycoside bond. The sugar chain unit may contain a lactated, sulfated sugar as the sugar constituent. In the sugar chain unit, 2.7 or more hydroxyl groups per 100 hydroxyl groups may be sulfated or sulfur elements may occupy 1.5% by weight or more of all of the elements.

In a biopolymer in accordance with embodiments of the present invention, the sacran may be obtained from Aphanothece Sacrum. For example, sacran may be obtained by protonating a polysaccharide extracted from Aphanothece sacrum, and by performing partial hydrolysis thereon. However, embodiments are not necessarily limited to sacran obtained (derived) from Aphanothece sacrum.

Ginipin may refer to methyl (1R,2R,6S)-2-hydroxy-9-(hydroxymethyl)-3-oxabicyclo[4.3.0]nona-4,8-diene-5-carboxylate. Ginipin is an aglycone derived from geniposide, an iridoid glycoside, which may be found in fruit of Gardenia jasminoides. Genipin can be prepared by oxidation followed by reduction and hydrolysis or by enzymatic hydrolysis of the parent compound geniposide, or, alternatively, racemic genipin can be prepared synthetically.

The term "genipin or an analogue or derivative thereof" refers to genipin, its derivatives, analogs and any stereoisomers or mixtures of stereoisomers of genipin. Thus, genipin, its derivatives, analogs, any stereoisomers, or mixtures of stereoisomers of genipin, or any combination thereof, can be used as the cross-linker referred to in embodiments of the present invention. Illustrative genipin analogues and derivatives include, but are not limited to, a compound represented by:

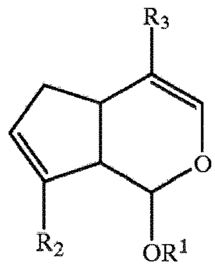

,

wherein $R_1$ represents lower alkyl, benzyl, or C1-C11 alkylcarbonyl which can be substituted with phenyl, phenoxy, pyridyl, t-butyl or thienyl, $R_2$ represents hydroxymethyl, formyl, acetyl, hydroxyiminomethyl, methoxyiminomethyl, lower alkylaminomethyl, acetylthiomethyl, mercaptomethyl, 2,2-dimethyl-1,3-dioxolan-5-ylmethyloxymethyl, 2,3-dihydroxypropyloxymethyl, 6-aminofuran-9-ylmethyl, 4-amino-2-hydroxy-5-methylpyrimidin-1-ylmethyl, 2,4-dihydroxy-5-methylpyrimidin-1-ylmethyl, 5-hydroxymethyl-1,3-oxathiolan-2-yl, or C1 -C11 alkylcarbonyloxymethyl which can be substituted with phenyl, phenoxy, pyridyl, t-butyl or thienyl, and $R_3$ represents methoxycarbonyl, formyl, hydroxyiminomethyl, methoxyiminomethyl, 4-methoxybenzyloxymethyl or acetyloxymethyl, provided that $R_3$ is not methoxycarbonyl when $R_1$ is lower alkyl, acetyl or t-butylcarbonyl and $R_2$ is hydroxymethyl, formyl, hydroxyiminomethyl, acetyloxymethyl or t-butylcarbonyloxymethyl, and $R_3$ is not acetyloxymethyl when $R_1$ is t-butylcarbonyl and $R_2$ is acetyloxymethyl, its pharmaceutically acceptable salt, or stereoisomer. Another illustrative genipin analogue or derivative may be represented by the following

formula:

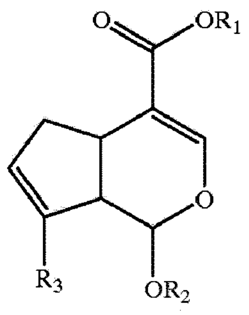

,

in which $R_1$ represents lower alkyl; $R_2$ represents lower alkyl, pyridylcarbonyl, benzyl or benzoyl; $R_3$ represents formyl, hydroxymethyl, azidomethyl, 1-hydroxyethyl, acetyl, methyl, hydroxy, pyridylcarbonyl, cyclopropyl, aminomethyl substituted or unsubstituted by (1,3-benzodioxolan-5-yl)carbonyl or 3,4,5-trimethoxybenzoyl, 1,3-benzodioxolan-5-yl, ureidomethyl substituted or unsubstituted by 3,4,5-trimethoxyphenyl or 2-chloro-6-methyl-3-pyridyl, thiomethyl substituted or unsubstituted by acetyl or 2-acetylamino2-ethoxycarbonyethyl, oxymethyl substituted or unsubstituted by benzoyl, pyridylcarbonyl or 3,4,5-trimethoxybenzoyl; provided that $R_3$ is not methyl formyl, hydroxymethyl, acetyl, methylaminomethyl, acetylthiomethyl, benzoyloxymethyl or pyridylcarbonyloxymethyl when $R_1$ is methyl, and its pharmaceutically acceptable salts, or stereoisomers.

Another illustrative genipin analogue or derivative may be represented by the following formula:

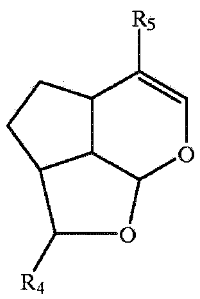

,

wherein $R_4$ represents lower alkoxy, benzyloxy, benzoyloxy, phenylthio, C1 ~ C12 alkanyloxy substituted or unsubstituted by t-butyl, phenyl, phenoxy, pyridyl or thienyl; $R_5$ represents methoxycarbonyl, formyl, hydroxyiminomethyl, methoxyimino-methyl, hydroxymethyl, phenylthiomethyl or acetylthiomethyl; provided that $R_5$ is not methoxycarbonyl when $R_4$ is acetyloxy; and its pharmaceutically acceptable salts, or stereoisomers.

Another illustrative genipin analogue or derivative may be represented by

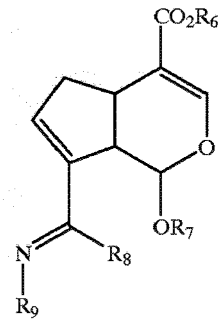

,

where $R_6$ represents hydrogen atom, lower alkyl or alkalimetal; $R_7$ represents lower alkyl or benzyl; $R_8$ represents hydrogen atom or lower alkyl; $R_9$ represents hydroxy, lower alkoxy, benzyloxy, nicotinoyloxy, isonicotinoyloxy, 2-pyridylmethoxy or hydroxycarbonylmethoxy; provided that $R_9$ is not hydroxy or methoxy when $R_6$ is methyl and $R_8$ is hydrogen

atom; and its pharmaceutically acceptable salts, or stereoisomers.

Another illustrative genipin analogue or derivative may be represented by

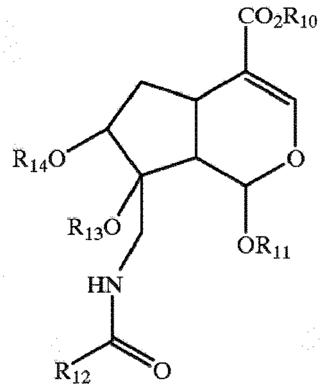

,

wherein $R_{10}$ represents lower alkyl; $R_{11}$ represents lower alkyl or benzyl; $R_{12}$ represents lower alkyl, pyridyl substituted or unsubstituted by halogen, pyridylamino substituted or unsubstituted by lower alkyl or halogen, 1,3-benzodioxolanyl; $R_{13}$ and $R_{14}$ each independently represent a hydrogen atom or join together to form isopropylidene; and its pharmaceutically acceptable salts, or stereoisomers.

Other illustrative genipin analogues and/or derivatives are disclosed in US 7,649,014, the content of which is incorporated herein by reference.

In a second aspect, the present invention relates to a method of producing a biopolymer, e.g. a biopolymer in accordance with embodiments of the first aspect of the present invention. The method comprises providing a polydeoxyribonucleotide (PDRN), or a derivative or modification thereof, having a molecular weight in the range of 400 000 Dalton (or 400 kDa) to 3 200 000 Dalton (or 3.2 MDa). The method comprises providing an anionic and ampholytic heteropolysaccharide having sulfate and carboxyl groups and a molecular weight in the range of 14 000 000 Dalton (or 14 MDa) and 16 000 000 Dalton (or 16 MDa). The method comprises crosslinking the polydeoxyribonucleotide and the heteropolysaccharide by genipin, a water soluble aglycon, or an analogue or derivative thereof.

A method in accordance with embodiments of the present invention may comprise dissolving PDRN matrix in deionized water to obtain a first solution of PDRN in the range of 0.005 wt% to 10wt%.

A method in accordance with embodiments of the present invention may comprise dissolving Sacran matrix in deionized water to obtain a second solution of Sacran in the range of 0.005 wt% to 10 wt%.

A method in accordance with embodiments of the present invention may comprise adding Genipin into a blended solution of said first solution and said second solution to obtain a solution of Genipin in the range of 0.05 wt% to 2.5 wt%, preferably 2.5 wt%. Thus a third solution of Genipin, Sacran and PDRN may be obtained by this blending.

In a method in accordance with embodiments of the present invention, the blending may comprise continuous stirring at a temperature in the range of 40°C to 50°C, e.g. 45°C, for at least 4 hours, e.g. 8h, or even longer, e.g. in the range of 6 hours to 24 hours.

A method in accordance with embodiments of the present invention may comprise dispersing the (third) solution of Genipin, PDRN and Sacran under magnetic stirring.

A method in accordance with embodiments of the present invention may comprise filtrating said (third) solution to eliminate air bubbles, e.g. after said step of dispersing.

A method in accordance with embodiments of the present invention may comprise crosslinking the (third) solution, e.g. in an oven, at a temperature in the range of 30°C to 45°C for at least 12 hours, e.g. preferably in the range of 35°C to 40°C, e.g. 37°C. Preferably, the crosslinking step comprises a heating step, e.g; at a substantially constant temperature, for at least 24 hours, e.g. 24 hours, 30 hours, 36 hours or even 48 hours.

A method in accordance with embodiments of the present invention may comprise gelling the crosslinked solution to obtain a gel comprising biopolymer scaffolds. For example, the crosslinked solution may be gelled at a temperature in the range of 0°C to 10°C, preferably in the range of 2°C to 6°C, e.g. 4°C, for 24 hours or longer.

A method in accordance with embodiments of the present invention may comprise lyophilizing the scaffolds.

Lyophilizing the scaffolds may comprise freezing the gel at a temperature, e.g. in the range of -40°C to - 100°C, e.g. in the range of -70°C to -90°C, e.g. -80°C, for at least 12 hours, e.g. 24 hours or even logner, and lyophilizing the frozen scaffolds e.g. for at least 12 hours, preferably at least 24 hours, e.g. for 48 hours or even longer.

In a method in accordance with embodiments of the present invention, the (third) solution may comprise a PDRN to Sacran ratio in the range of 70 : 30 to 30 : 70, preferably 60 : 40, e.g. 70 : 30, 60 : 40, 50 : 50, 40 : 60 or 30 :

70 (embodiments including any intermediate value).

In a third aspect, the present invention relates to a composition comprising the biopolymer in accordance with embodiments of the first aspect of the present invention for use in a biomedical, cosmetic, food or agricultural product.

In a composition in accordance with embodiments of the present invention, the biopolymer may be used as a carrier for direct or sustained release of an active pharmaceutical ingredient.

In a composition in accordance with embodiments of the present invention, the active pharmaceutical ingredient may be an epidermal growth factor.

In a fourth aspect, the present invention relates to a pharmaceutical comprising the biopolymer in accordance with embodiments of the first aspect of the present invention for treating inflammation and/or impaired wound healing.

In a fifth aspect, the present invention relates to the use of a composition in accordance with embodiments of the third aspect of the present invention or the pharmaceutical in accordance with embodiments of the fourth aspect of the present invention for the treatment of inflammation and/or impaired wound healing, e.g. for the treatment of a skin inflammatory disease.

The independent and dependent claims describe specific and preferred features of the invention. Features of the dependent claims can be combined with features of the independent claims and with features of other dependent claims as deemed appropriate, and not necessarily only as explicitly stated in the claims.

**Brief description of the drawings**

FIG. 1 shows FT-IR spectroscopy analysis of (A) PDRN, (B) Sacran, (C) noncrosslinked PDRN/Sacran scaffold, (D) PDRN/Sacran scaffolds crosslinked by genipin, (E) PDRN/Sacran scaffolds crosslinked by glutaraldehyde, and (F) PDRN/Sacran scaffolds crosslinked by EDC, respectively, for illustrating embodiments of the present invention.

FIG. 2 shows SEM micrography analysis for illustrating the morphology of PDRN/Sacran scaffolds which were crosslinked by (A) Genipin, (B) Glutaraldehyde, and (C) EDC, for illustrating embodiments of the present invention.

FIG. 3 shows DTG curves of the uncrosslinked and crosslinked PDRN/Sacran scaffolds to illustrate that the pyrolysis of each component occurs separately, for illustrating embodiments of the present invention.

FIG. 4 shows the various degrees of swelling as a function of time for uncrosslinked PDRN/Sacran film samples, for illustrating embodiments of the present invention.

FIG. 5 shows the various degrees of dissolution as a function of time for uncrosslinked PDRN/Sacran film samples, for illustrating embodiments of the present invention.

FIG. 6 shows, respectively, the swelling (a) and dissolution (b) degrees of the crosslinked Sacran film samples as function of the Genipin amount after 24h, for illustrating embodiments of the present invention.

FIG. 7 shows, respectively, the swelling (a) and dissolution (b) degrees of the crosslinked PDRN/Sacran film samples after 24h, for illustrating embodiments of the present invention.

FIG. 8 shows the elastic moduli of the Genipin-crosslinked film samples in a dry state, as a function of Sacran content and Genipin amount, for illustrating embodiments of the present invention.

FIG. 9 shows the creep and creep-recovery curves for PDRN/Sacran - 0.5 Genipin film samples.

FIG. 10 shows cell density data for the cast film samples crosslinked with a) 0.5% (w/w) and b) 2.5% (w/w) Genipin at 4, 24 and 48 h, as compared to a Sacran control layer, for illustrating embodiments of the present invention.

FIG. 11 shows a schematic representation and actual images of wound generation and implants, for illustrating embodiments of the present invention. The images show the template marks (A) on a Wistar rat, the created dorsal excision wound (B), and the blank or EGF-loaded hydrogel implant (C). The schematic shows the steps of (D) wound excision (25 mm x 25 mm x 1 mm), (E) covering of wounds with hydrogels (blank / loaded vs. control) and (F) determining wound parameters over an 8-day period (sacrifices at day 4 and 8).

FIG. 12 shows a histological evaluation of newly formed (a) and granulated (b) tissue on day 8 (images are representative of three biological replicates), and a comparison (c) of the granulation thickness in samples, for illustrating embodiments of the present invention.

FIG. 13 shows degrees of re-epithelialization in rat groups compared to control on days 4 and 8, for illustrating embodiments of the present invention.

FIG. 14 shows a histological evaluation of epithelial tissue regenaration in rat groups compared to control on days 4 and 8, for illustrating embodiments of the present invention.

FIG. 15 shows macroscopic imaging of the wound appearances in rat groups on days 0, 4, and 8, for illustrating embodiments of the present invention.

FIG. 16 shows (a) the percentage of wound closure in rat groups on days 4, and 8, and (b) the residual wound surface of treated rats in comparison with day 0, for illustrating embodiments of the present invention.

FIG. 17 shows an inflammatory response evaluation by staining of tissue sections with Hoescht 33528 and Iba1 in

different rat groups on day 4 and 8, for illustrating embodiments of the present invention.

The drawings are schematic and not limiting. Elements in the drawings are not necessarily represented on scale. The present invention is not necessarily limited to the specific embodiments of the present invention as shown in the drawings.

**Detailed description of embodiments**

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

The embodiments of the present invention are specifically described below with reference to the embodiments, so as to facilitate the understanding of the present invention by those skilled in the art. It should be noted that the embodiments are only used for further explanation of the present invention, cannot be understood to limit the protection scope of the present invention, and a person skilled in the art will recognize that the protection scope of the present invention can be better understood by those skilled in the art, the non-essential improvement and adjustment made by the method disclosed by the invention should still be within the protection scope of the invention.

The raw materials are commercially available products; the process steps or the preparation method which are also not always described in detail, are process steps or preparation methods which are all known by those skilled in the art. Meanwhile, raw materials being used are not always described in detail.

In this detailed description, various specific details are presented. Embodiments of the present invention can be carried out without these specific details. Furthermore, well-known features, elements and/or steps are not necessarily described in detail for the sake of clarity and conciseness of the present disclosure.

The present invention relates to a novel engineered biodegradable biopolymer. It comprises the crosslinking of two biodegradable biopolymers: the first being PDRN, or a derivative or modification thereof, the other being Sacran, or a derivative or modification thereof, with Genipin functioning as a natural crosslinking agent.

Other possible formations from crosslinking Sacran to another moiety (such as, but not limited to, hyaluronic acid, chitosan, cellulose, heparin, collagen, elastin, heparosan, carrageenan, starches, alginate, gelatin, or derivatives thereof), or by using another synthetic, or natural crosslinking agents (for example, but not limited to, glutaraldehyde, tripolyphosphate, ethylene glycol, diglycidyl ether and diisocyanate, Proanthocyanidin, Chitosan, Epigallocatechin-3-gallate, Low dose riboflavin/UVA-activated riboflavin, hesperidin, Galardin, or derivatives thereof) are also within the scope of this present invention.

In accordance with embodiments of the present invention, there is provided the process of obtaining the Genipin-crosslinked PDRN/Sacran scaffolds, which have shown to exhibit many of the desired characteristics important for optimal therapeutic function. In particular, the Genipin-crosslinked PRDN-Sacran scaffolds with strong muco-adhesive, anti-inflammatory, angiogenic, and drug loading capability may be suitable in therapeutic applications that involve many inflammatory diseases and tissue engineering applications.

Also in accordance with embodiments of the present invention, is the application of the Genipin-crosslinked PDRN/sacran scaffolds serving as hydrogel implants or in the form of dressings for improved wound healing/closure. Such implants or dressings can also be loaded with APIs in order to improve the healing rate of the wound. The particular use of EGF in combination with the Genipin-crosslinked PDRN/Sacran scaffolds, Genipin-crosslinked PDRN scaffolds, or Genipin-crosslinked Sacran scaffolds, is also subject to present invention and further explained in detail further hereunder. The use of any other API in combination with the Genipin-crosslinked PDRN/Sacran scaffolds, Genipin-crosslinked PDRN scaffolds, or Genipin-crosslinked Sacran scaffolds, is also subject to present invention.

The use of PDRN-Genipin-Sacran in biomedical, cosmetic, food, and agricultural applications are also within the scope of this invention.

The use of PDRN and Sacran combined in a mixture, without being crosslinked, in all biomedical, cosmetic, food, and agricultural applications are also within the scope of this invention.

The use of PDRN-Genipin crosslinked to any other biological material, in all biomedical, cosmetic, food, and agricultural applications are also within the scope of this invention.

The use of Sacran-Genipin crosslinked to any other biological material, in all biomedical, cosmetic, food, and agricultural applications are also within the scope of this invention.

The use of PDRN-Genipin-Sacran crosslinked to any other biological material, in all biomedical, cosmetic, food, and agricultural applications are also within the scope of this invention.

According to embodiments of the present invention, the PDRN moiety of the PDRN-Genipin-Sacran conjugated polymer may be a mixture of highly polymerized deoxyribonucleotides or polydeoxyribonucleotides (hererinafter referred to as "PDRN"). PDRN in preferably in the form of a sodium salt by which non-denaturing techniques ensures a perfect protection of the molecular structure of the DNA (in particular preserves the DNA in its double-stranded form), and thus preserves its physiological activity. Other preferable forms may include iron, chromium, magnesium, and manganese salts. Preferably, the highly polymerized DNA of the composition (PDRN) of the invention may be obtained by the implementation of non-denaturing processes. PDRN is known for its tissue regeneration activity at cellular level through the stimulation of the production of white blood cells, lymphocytes and neutrophils. It may be obtained from naturally-occurring sources such as animal sources, and in particular from the reproduction cells of certain aquatic animals, particularly such as the testes, milt or roe sacs of rainbow/steelhead trout (Oncorhynchus mykiss). Other species also include: cutthroat trout (Oncorhynchus clarki), pink salmon (Oncorhynchus gorbuscha), chum salmon (Oncorhynchus keta), coho salmon (Oncorhynchus kisutch), masu/cherry salmon (Oncorhynchus masou), sockeye/kokanee (Oncorhynchus nerka), and chinook salmon (Oncorhynchus tshawytscha). Although the milt from acispencer, huso, scaphirhynchus, and pseudoscapirhynchus sturgeon species can also be used, due to them being critically endangered, their use may be less preferred.

PDRN may be extracted and purified at high temperature, a procedure that allows to recover a >95% pure active substance with inactivated proteins and peptides. This latter may contribute to the safety of the product and avoid immunological side effects. Spermatozoa may be preferable as source material to provide highly purified DNA without risk of impurity such as peptides, proteins and lipids which can remain from the somatic cells.

Such process may relate to extraction and purification under high temperature: I) enzyme lysis of the protein organic matrix; II) clarifying filtration of the resulting solution; III) precipitation with quaternary ammonium salts; IV) decomplexing of the precipitate; V) molecular selection using column chromatography; VI) precipitation of the eluate using alcohol until a negative protein reaction is indicated in the eluate, and VII) recovering of the purified biological material in the eluate. "Highly polymerized DNA" may refer to a DNA having a molecular mass of between 1000 and 5000 kDa, preferably between 1500 and 4000 kDa, preferably between 1750 and 3000 kDa, and preferably of 2400 kDa.

The nucleotide composition of the DNA extracted from rainbow/steelhead trout (Oncorhynchus mykiss) testes is as follows:

| | |
|---|---|
| Adenine: | 29.7 $\pm$ 0.08 |
| Thymine: | 29.1 $\pm$ 0.09 |
| Guanine: | 20.8 $\pm$ 0.08 |
| Cytosine | 20.4 $\pm$ 0.07 |

A native DNA sodium salt may be used in accordance with embodiments of the invention; e.g. having a specific molecular weight between 200x104 daltons to 240x104 daltons, e.g. and having a hyperchromicity effect (G): G 41%.

As an example, 0.20 g of the preparation may be placed into a measured 30 ml retort, 0.1% sodium chloride solution may be added, the preparation may be dissolved and the volume may be brought up to the mark. 1 ml of the solution may be placed into a measured 250 ml retort, the volume may be brought up to the mark with 0.2% sodium chloride solution. Absorption of the received solution ($D_{260}$) may be measured.

10 ml of solution may be placed into a retort with thin section, 10 ml of 15% chloric acid solution, closed and boiled on a water bath for 25 min. The solution may be cooled own to the normal temperature, transferred by quantity into a measured 200 ml retort, and the volume may be brought up to the mark with water. 10 ml of this solution may be placed into a measured 100 ml retort and the solution volume may be brought up to the mark with water. Absorption of the received solution ($D^1_{260}$) may be measured.

The PDRN moiety of the total PDRN-Genipin-Sacran may suggest a wider pleiotropic modality and anti-inflammatory action versus PDRN in its native state, due to the crosslinking with highly ampholytic sulfated Sacran. Indeed, the studies performed by the authors of the present invention show a significant reduction of the serum levels of a number of inflammatory mediators: Th-1 and Th-2 cytokines such as Interferon-gamma (IFN-y), Tumor necrosis factor alpha (TNF-$\alpha$) (although also downregulated by PDRN), Interleukin 4 (IL-4), Interleukin 5 (IL-5), Eotaxin, and Monocyte chemoattractant protein-1 (MCP-1).

In vitro and in vivo experiments have suggested that PDRN most relevant mechanism of action is the engagement of adenosine $A_{2A}$ receptors, which are responsible for sending out a regeneration signal to secrete various growth factors. It is known as a component that promotes capillary production by stimulation of vascular endothelial growth factor (VEGF), thus improving blood circulation, anti-inflammatory action and prevention of capillary leakage. By stimulating an even wider pleiotropic pathway, PDRN increases anti-inflammatory Interleukin-10 factor (IL-10), and decreases pro-inflammatory cytokines such as Interleukin-6 (IL-6), Tumor Necrosis Factor alpha (TNF-a), and High mobility group protein 1 (HMGB1) which are suggested to contribute to the pathogenesis of various chronic inflammatory and autoimmune diseases, and cancer. High serum levels are found in several inflammatory events including but not limited to; sepsis, periodontitis, gingivitis rheumatoid arthritis, artherosclerosis, chronic kidney disease, systemic lupus erythematosus (SLE). Furthermore PDRN positively stimulates the secretion of Epidermal growth factor, Fibroblast growth factor, and Insulin growth factor, which are key mediators in fibroblast, osteoblast, and chondrocyte proliferation, and differentiation.

Besides engaging the $A_{2A}$ receptor, PDRN also provides nucleosides and nucleotides for the so-called "salvage pathway." The binding to adenosine $A_{2A}$ receptors however, is a unique property of PDRN and seems to be linked to DNA origin, molecular weight and manufacturing process. In this context, PDRN represents a new advancement in pharmacotherapy and may be particularly useful towards creating modified biodegradable biopolymers with improved pharmacological action.

In terms of the chemical structure, PDRN may be composed of low-molecular weight DNA and may be a linear polymer comprising 50-2,000 base pairs. PDRN is degraded by active membrane enzymes to serve as a source of purine and pyrimidine deoxynucleosides and deoxyribonucleotides, which increase cellular proliferation and activity in different tissues. The structure of an illustrative polydeoxyribonucleotide (PDRN) segment held by phosphodiester bonds is shown hereunder:

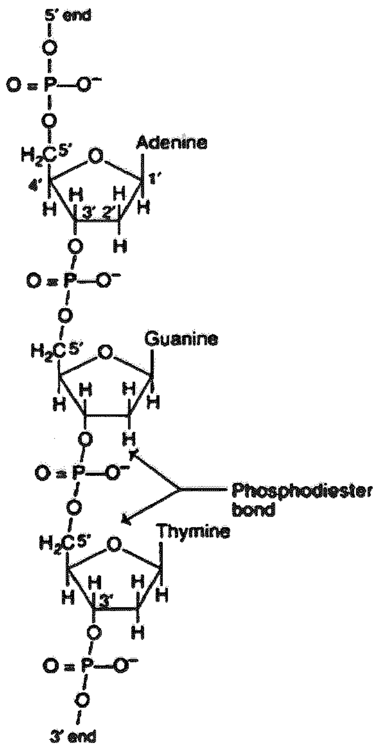

Genipin may be a preferred water-soluble bi-functional natural crosslinking agent of choice. This Genipin may relate to a 226 Da aglycone derived from geniposide, an iridoid glucoside. Geniposide is present in the fruit of Gardenia jasminoides, a common flower in many parts of Asia. It is widely used in herbal medicine, and the dark blue pigments obtained by its spontaneous reaction with amino acids or proteins have been used in the fabrication of food dyes. Other applications involve the preparation of gelatin capsules and the immobilization of enzymes. At acidic and neutral pH, Genipin reacts with primary and tertiary amines of biopolymers and forms mono- up to tetramer crosslinks. With increasingly basic conditions, Genipin further undergoes ring-opening self-polymerization with increasing polymer length prior to binding to primary or tertiary amines. With increasing polymer length (~4-88-mers), amine reactions with Genipin slows, which leads to less reduced enzyme digestibility and swelling by Genipin. The feasibility and benefit of employing Genipin as an alternative (Genipin has a low acute toxicity, with LD50 i.v. 382 mg/k in mice) to higher toxicity crosslinkers like glutaraldehyde has been demonstrated in a range of applications, including heart valves, pericardial patches, conduits for nerve growth guidance, scaffolds for tissue-engineered cartilage, and decellularized tracheal transplantation, and as a more general application to augment the strength and degradation properties of collagen-based gels. The authors of the present invention have found that genipin can be used as a suitable crosslinking agent for the formation of PDRN-Sacran hydrogels.

An illustrative structure of Genipin is presented hereunder:

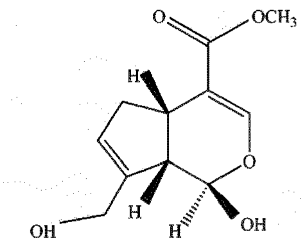

Although the structure presented above shows the natural configuration of genipin, any stereoisomer or mixture of stereoisomers of genipin as shown later may be used as a crosslinking reagent, in accordance with the present invention.

Sacran may refer to an anionic and ampholytic heteropolysaccharide with sulfate and carboxyl groups. It may be extracted from the Japanese indigenous cyanobacterium Aphanothece sacrum, which is mass-aquacultured in rivers with a high ionic concentration and possesses plenty of jelly-like extracellular matrix with high water content (97.5 - 98.3%). Its structure can be very complex due to many kinds of sugar residues (galactose, glucose, mannose, xylose, rhamnose, fucose, galacturonic acid, and glucuronic acid) and may contain traces of alaline, galactosamine, uronic acids (which uronic acids have yet to be determined chemically), and muramic acid; 11% of its monosaccharides may contain a sulfate group of which 22% may contain a carboxyl group. That is, the aqueous solution of sacran is a polyelectrolyte.

Due to its electric charges on the chain, sacran changes its conformation depending on the sacran concentration, e.g., helix transition concentration, where the sacran chain change from a random coil to double helix occurs at 0.09 wt.% and gelation concentration, where sacran shows a transition from liquid to weak gel at 0.25 wt.%.

Sacran shows various unique phenomena such as high swelling and anisotropic swelling behaviors, liquid crystalline (LC) behavior, ion adsorption behavior, and anisotropic diffusion behavior in highly ordered sacran films. The retention capacity for uncross-linked sacran exceeds higher than 6000 mL/g for pure water and 2700 mL/g for NaCl aqueous solution. Similar high swelling behaviors were also found for cross-linked sacran hydrogels (e.g., 6100 mL/g for pure water and 530 mL/g for 0.9 wt.% for saline solution).

These high-swelling behaviors might originate from the large mesh consisting of extremely long chains of sacran or macroscopic LC domains with millimeter to several centimeter scales in which sacran chains are aligned by self-organization. For ion adsorption, sacran shows a feature that the adsorption ratio for sacran against trivalent neodymium ions (Nd3+) is twice that for sodium alginate.

Thus, sacran demonstrates various unique phenomena originating from the feature of super megamolecules. Anomalous property can also be seen in the rheological properties of sacran; that is, the viscosity increases over time when a steady shear with low shear rates is applied.

An illustrative structure of sacran chains, where the sequences in monosaccharide triads, pattern of glycoside linkages, steric conformations of hydroxyls or other substitutes are tentative, is pictured hereunder:

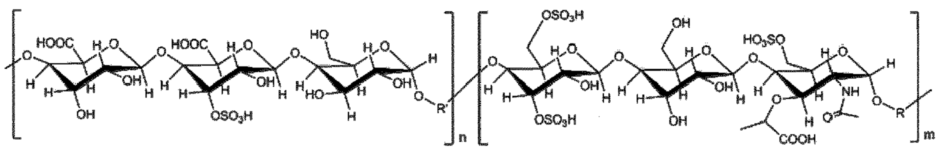

R, R', R":

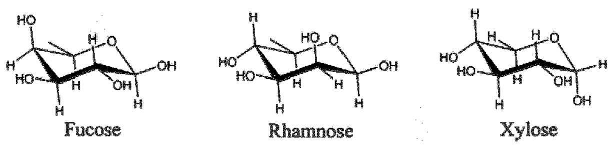

Fucose Rhamnose Xylose

<u>Crosslinking reaction</u>

The reaction mechanism is a two-step process requiring two distinct reactions in an aqueous solution. The first reaction results in the heterocyclic linking of Genipin and occurs when the ring-opening reaction of Genipin undergoes a nucleophilic attack by the carbonyl & amine groups on the purine and pyrimidine rings of Adenine and Guanine (as purines), and Cytosine (as pyrimidine) in PDRN; and the many carboxyl groups present in Sacran. The second, slower, reaction occurs when the ester group on genipin undergoes a nucleophilic substitution of the ester group of genipin to form a secondary carbonyl and amide link with PDRN and carboxyl link with Sacran.

The resultant covalent crosslinks between the primary and secondary amine residues leave very minimal

residual toxicity. In addition, the resultant crosslinked material can form ionic complexes with charged molecules and therefore may aid in sequestering proteins as well.

An illustrative genipin cross-linked PDRN-Sacran hydrogel may comprise the following raw materials in percentage by weight: 0.05wt%-2.5wt% of genipin, 1wt%-10wt% of matrix (PDRN-Sacran) and the balance of water. The composition of the matrix can be divided into the following ratios (for example): PDRN 70: Sacran 30, PDRN 60: Sacran 40, PDRN 50: Sacran 50, PDRN 40: Sacran 60, PDRN 30: Sacran 70. By carrying out crosslinking by virtue of optimized genipin concentration, the obtained biogel has the properties of low toxicity, good water-absorbing property and mechanical property and the like. The resulted ratio in examples relating to the present invention is PDRN 60: Sacran 40, yet is merely illustrative and functions merely as an example of many variations in composition in accordance with embodiments.

Example of preparation of PDRN-Genipin-Sacran crosslinked biogel

As an example, 0.05 grams of PDRN was dissolved in deionized water (100ml), while 0.005 grams of Sacran was dissolved in deionized water (100ml). Both solutions were blended together under continuous stirring at 45°C for 8h. Different concentrations of genipin (0.5% or 2.5% (w/w)) were added into the mixture and dispersed with magnetic stirring. The solutions were filtrated twice to eliminate air bubbles and poured into 35 mm Petri dishes, which were put in a constant temperature oven at 37°C to crosslink for 24 h, after that in a freezer at 4°C to gel for 24 h, followed by an ultra cold freezer at -80°C to pre-freeze for 24 h respectively. The frozen scaffolds were lyophilized for 48h. Non-crosslinked PDRN/Sacran scaffolds were prepared without genipin using the same method. The scaffolds were balanced with $Na_2HPO_4$ solution (0.1M) and rinsed for 24 h with deionized water. Finally, the scaffolds were pre-freezing at -80°C for 24 h and then lyophilizing for 48h.

As a further control group, the PDRN-Sacran scaffolds crosslinked by glutaraldehyde and EDC were prepared according to methods known in the art. Glutaraldehyde is the most commonly used synthetic crosslinker, but it is poisonous and seriously impairs biocompatibility of crosslinked materials. EDC is a water soluble and non-toxic crosslinker, but the degree of crosslinking and uniformity is rather low.

The physico-chemical properties of the blends were analyzed (using thermogravimetric analysis, TGA; Fourier transform infrared-attenuated total reflectance spectroscopy, FTIR-ATR; scanning electron microscopy, SEM), as well as the functional characteristics in view of their potential application in tissue engineering (mouse fibroblast culture tests). Mechanical properties (stress-strain and creep tests) of crosslinked samples were also measured in a dry state by means of an apparatus based on an isotonic force transducer. Efficacy of genipin-crosslinking was evaluated by means of swelling and dissolution measurements carried out in media simulating physiological conditions. Fibroblasts adhesion tests performed on crosslinked cast films showed the biocompatibility of genipin-crosslinked samples.

Experimental results

FT-IR spectra were recorded on a SmartSpec 3000 spectrophotometer (Bio-Rad) in the wave number range 4000-400 $cm^{-1}$ using KBr pellets.

The scaffold samples (a total of 2% w/w) were mixed with potassium bromide. The mixtures were then homogenized into powders using a BeadBug 6 Position Homogenizer (Thomas scientific). The disks were compressed for scanning. At a minimum, each sample was assessed in triplicate. For SEM micrograph analysis, the scaffolds were cut into small identical samples using a surgical knife blade. The samples were all attached to aluminum stubs with double-sided sticky tabs while coated with gold for their analysis. With a JSM-F100 Field Emission Scanning Electron Microscope (Jeol). The pore dimensions of the genipin, glutaraldehyde and EDC crosslinked PDRN/Sacran scaffolds were measured by ImageJ software.

The FT-IR spectroscopy analysis in FIG. 1 shows the FT-IR spectra of (A) PDRN, (B) Sacran, (C) noncrosslinked PDRN/Sacran scaffold, (D) PDRN/Sacran scaffolds crosslinked by genipin, (E) PDRN/Sacran scaffolds crosslinked by glutaraldehyde, and (F) PDRN/Sacran scaffolds crosslinked by EDC, respectively. PDRN spectra showed that the peak appearance at 3340 $cm^{-1}$ is partially overlapped of amine and hydroxyl group stretching vibration. The characteristic peak of primary amine was the absorption band at 1659 $cm^{-1}$. Characteristic of CAN stretching vibration was the peak of 1079 $cm^{-1}$. The characteristic peaks of Sacran stretching vibration were mainly at 1642 $cm^{-1}$ for amide I (C--O stretching vibration) and at 1549 $cm^{-1}$ for amide II (N-H bending vibration). The absorption peak of the Genipin crosslinked PDRN/Sacran scaffold decreased at 1553 $cm^{-1}$ compared with the non-crosslinked PDRN/Sacran scaffold. It is suggested that nucleophilic attack by the carbonyl & amine groups of PDRN/Sacran on the olefinic carbon atom at C-3 of genipin followed is by the opening of the dihydropyran ring and formed a tertiary amine. The shift at 1657 $cm^{-1}$ suggests the formation of heterocyclic amine after crosslinking. This suggests that the partially amide group on PDRN/Sacran scaffolds transforms into heterocyclic amines of Genipin. The increasing intensity of absorption peak at 1658 $cm^{-1}$ (C--N and C--O stretching vibration) suggests that glutaraldehyde as crosslinker can react with amide group on PDRN/Sacran scaffolds

and transforms into the structure via the Schiff base reaction. The increasing of the intensity of absorption band at 1658 $cm^{-1}$ and 1554 $cm^{-1}$ for the correlating spectrum of EDC crosslinked scaffold suggests that EDC could translate amino group into amide. These results confirmed that the PDRN/Sacran scaffolds were crosslinked by different crosslinkers.

The morphology of the scaffolds which were crosslinked by different crosslinkers is shown by SEM micrograph analysis in FIG. 2. Open macro-pore structures and general structures show better interconnection with each other. Furthermore, the sufficient porosity of the scaffolds support an ideal microenvironment for API loading. The pore dimensions of the genipin (A), glutaraldehyde (B) and EDC (C) crosslinked chitosan/gelatin scaffolds were 244 ± 34 μm, 163 ± 57 μm, and 164 ± 43 μm, respectively. The pores of the glutaraldehyde and EDC crosslinked PDRN/Sacran scaffold however, shows a high degree of inhomogeneity. Compared with the glutaraldehyde and EDC crosslinked PDNR/Sacran scaffold, the Genipin crosslinked PDRN/Sacran scaffold shows bigger pores and more uniform and homogenous pore distribution.

A thermogravimetric analysis was performed using a TGA 5500 thermogravimetic analyzer (TA instruments) where thermal degradation was measured under nitrogen atmosphere. The experiments were performed at a 5°C/min heating rate in the 40 - 750°C temperature range.

Two weight loss phenomena are displayed as decomposition traces of both crosslinked PDRN/Sacran, and uncrosslinked but blended PDRN/Sacran samples show water evaporation at 50-150°C and polymer pyrolysis in the 200-500°C temperature range. The maximum degradation rate temperatures ($T_d$s) of all samples derived from the corresponding PDRN/Sacran 60/40 samples are shown in the table hereinbelow.

| Samples | $T_d$ (°C) | | |
|---|---|---|---|
| | Uncrosslinked | Crosslinked | |
| | | 0.5% (w/w) | 2% (w/w) |
| PDRN | 275 - 296 | 296.7 | 293 |
| PDRN/Sacran 60/40 | 280; 339* | 283.5; 342* | 290; 332 |
| Sacran | 334 | 336 | 279*; 324 |

Values marked with an asterisk (*) indicate the temperature of the shoulder of the recorded degradation peaks.

The Sacran sample is more stable than the PDRN sample, both in a crosslinked an uncrosslinked state. The DTG curves in FIG. 3 of the uncrosslinked and crosslinked PDRN/Sacran scaffolds show that the pyrolysis of each component occurs separately; pyrolysis peaks of the scaffold components are partially overlapped with values of $T_d$s similar to those of the native components (PDRN and Sacran respectively). The values of $T_d$ do not change significantly upon crosslinking. These results confirm that the use of 0.5% and 2% (w/w) Genipin do not significantly affect heat resistance of crosslinked samples.

Swelling and dissolution tests were performed on uncrosslinked and crosslinked PDRN/Sacran film samples (measurements: 2 x 2 $cm^2$). The film samples were weighed and then put in phosphate buffered saline solution (Gibco PBS, Thermo Fisher) with pH 7.4 at 37.5°C. At various times (1, 3, 6, 24 and 48h), the swollen film samples were taken out of the PBS solution and dried superficially by contact with filter paper and weighed again.

The swelling percentage was calculated as $\%S_w = [(W_s - W_i)/W_i] \times 100$, where $W_s$ and $W_i$ are the film sample weights before and after swelling, respectively. Samples were weighed again after drying them at 37.5°C for 48 hours in a vented oven.

Solubility percentages were calculated as $\%S = [(Wi-W_d)/W_i] \times 100$, where $W_d$ is the dried film sample weight after dissolution testing. Each test consisted of measurements in three replicates and the results were expressed as average values.

FIG. 4 and 5 show the various degrees of swelling and dissolution as a function of time for uncrosslinked PDRN/Sacran film samples, respectively. The swelling and dissolution behavior of uncrosslinked film samples increases with increasing Sacran content: Sacran and PDRN/Sacran 20/80 film samples dissolve within 1h. The swelling degrees of PDRN/Sacran 60/40 and 40/60 blends display a maximum at 3h (about 220 wt.%) and at 1h (250 wt.%), respectively. The abscissa of the maximum of swelling data shifts towards lower times with increasing the Sacran amount, due to the lower stability of Sacran-rich samples. The dissolution degrees of PDRN/Sacran 60/40 and 40/60 increase with increasing time, reaching a value at 56 wt.% and 84 wt.%, respectively after 24h ($p < 0.05$ PDRN/Sacran 60/40 vs. PDRN/Sacran 40/60).

By increasing the PDRN amount, the stability of PDRN/Sacran samples in aqueous media increases, due to the lesser solubility of PDRN in media having a higher pH than 6.5. The differences between the degrees of PDRN and PDRN/Sacran 60/40 are not statistically significant after 24-48 h ($p > 0.05$).

The swelling and dissolution degrees of the crosslinked Sacran film samples as function of the Genipin amount after 24h are shown in FIG. 6a and 6b, respectively. The film samples completely dissolve at a shorter time than 24h for lower Genipin contents than 2% (w/w). By increasing the Genipin amount from 2.0% (w/w) to 2.5% (w/w), the swelling

and dissolution degrees of Sacran decrease and are kept substantially unchanged for higher Genipin contents than 2.5% (w/w).

This result confirms that the water stability of Sacran (and therefore also its crosslinking degree) does not vary appreciably using higher Genipin contents than 2.5% (w/w).

The swelling and dissolution degrees of the crosslinked PDRN/Sacran film samples after 24h, respectively are shown in FIG. 7a and 7b. By increasing the Genipin content from 0.5% (w/w) up to 2.5% (w/w) ($p < 0.05$), the swelling degree of the blends decrease, whereas that of PDRN increases with increasing the Genipin amount ($p < 0.05$).

These findings can be attributed to the prevalence of oligomerisation reactions of Genipin on pure PDRN, due to the more acidic characteristics of the reaction environment. A higher degree of Genipin oligomerisation leads to a porous network with higher swelling properties. Independently from the Genipin amount, the dissolution degree of crosslinked PDRN film samples are close to 0 wt.% after 24h. By increasing the Sacran amount, the dissolution degrees for PDRN/Sacran - 0.5 Genipin increase, and are found to be much higher than those of the corresponding compositions crosslinked with 2.5 (w/w) Genipin ($p < 0.05$). With respect to the film samples crosslinked with 0.5 (w/w) Genipin, the dissolution degree of the PDRN/Sacran-2.5 GP film samples have a lower dependence on the Sacran amount. The various differences between the dissolution degrees of PDRN/Sacran 60/40 - 2.5 Genipin, PDRN/Sacran 20/80 - 2.5 Genipin and Sacran - 2.5 Genipin are not statistically significant ($p > 0.05$). In fact, Sacran - 2.5 Genipin only loses 14.6 wt. % after 24h immersion in PBS.

These results conclude that crosslinking treatment using 2.0 to 2.5 wt % Genipin allows the obtainment of film samples with higher water stability.

To measure the tensile properties of the Genipin-crosslinked PDRN/Sacran films, an isotonic transducer was used with an applied force at 1mN resolution.

The tensile properties of GP-crosslinked CS/G films were measured using an isotonic transducer (model MLT0015, Harvard Apparatus), where the applied force has a resolution of 1 mN. The test specimens were dry cast films with 2cm in length, 1 cm in width and a height of around 150 μm. An optical microscope (Bioscience 40-1000x Trino, Bresser) equipped with a digital camera, was used to record images of each scaffold and to obtain the correlating data on initial dimensions and geometry. Along the length of the film samples, traction force was applied. For each composition, three specimens were tested. From the slope of the initial linear portion of the stress-strain curve, the Young's modulus of each sample was calculated. On identical film samples, creep measurements were performed, using the same instrument as for the tensile tests. The load corresponding to 10% of the maximum elastic deformation of the specimens was "instantaneously" applied to each film samples and then kept constant for a period of 1h, during the developing strain y(t) was measured. The recoverable strain was monitored for further 60min (creep recovery measurement) after load removal.

The elastic moduli of the Genipin-crosslinked film samples in a dry state, as a function of Sacran content and Genipin amount are shown in FIG. 8. The elastic moduli of the samples increases with increasing the Genipin amount at each composition. The film samples are found to be stiffer due to a higher crosslinking density.

The differences between the elastic modulus of the film samples crosslinked with 0.5 wt % and 2.5 wt % Genipin amounts, however, were only statistically significant for samples that contain 80 wt % Sacran. For blend crosslinked with the same Genipin amount, on the other hand, the elastic modulus decreases with increasing the Sacran content. The creep and creep-recovery curves for PDRN/Sacran - 0.5 Genipin film samples normalised with respect to the applied stress, i.e. compliance curves as exemplary of the creep and creep-recovery behavior of Genipin-crosslinked film samples, are shown in FIG. 9. The creep and creep-recovery curves for PDRN/Sacran - 2.5 Genipin blends are very close to those of PDRN/Sacran - 0.5 Genipin blends and therefore they are not reported in detail. By increasing the Sacran amount, instantaneous compliance of the samples increased. This behavior is in agreement with the Young Modulus measurements which show an increasing stiffness of the film samples when increasing the PDRN amount. Especially for Sacran rich samples, compliance slightly increases with time. Due to the viscoelastic properties of the materials, in particular after a very short transient response, the compliance settles to an approximately constant value, which is only slightly higher than the instantaneous compliance. The load removal causes a quick decrease of the strain, which can be related tot he elastic response of the film samples.

On cast films, cell adhesion and proliferation tests were performed, using NIH-3T3 mouse fibroblast cells. The cells were cultured in Dulbecco's modified Eagle's medium (DMEM, Thermo Fisher) with high glucose, 15% fetal bovine serum (FBS, Sigma-Aldrich), 2% glutamine (Sigma-Aldrich), 250 U/mL of Penicillin (Sigma-Aldrich) and 200μg/mL of streptomycin (Sigma-Aldrich). 120,000 cells/mL were the typical seeding densities. The film samples were placed in a 24-well plate (Thermo Fisher), after a twice washing and sterilization cycle. The film samples were precoated with 0.1 wt % poly-l-lysine in phosphate buffered saline (Sigma-Aldrich). To each well, 1.5ml of adhesion factor solution was added and left in incubation for 1h at 37.5°C and finally aspired. Cell adhesion measurement was performed on three film samples for each time (4, 24, 48h). The culture medium was removed and substrates with attached cells were rinsed with phosphate buffered saline (Sigma-Aldrich) after each culture time. The attached cells were fixed by addition of 4.5% (v/v) formaldehyde (Sigma-Aldrich) solution in phosphate buffered saline for 10 min. and then stained with Coomassie

Blue (Thermo Fisher) solution for 10 min. Optical microscopy (Bioscience 40-1000x Trino, Bresser) was used to analyze the film samples for the ratio between the number of cells on the polymeric structures, and as an index of cell density, the total area of the polymer substrate was calculated. For comparison to evaluate the effect of scaffold topography on cell proliferation, both cells of a reference sample and cells seeded onto the scaffolds were examined. Poly-l-lysine was selected as a control, respectively for the test on mouse fibroblasts.

FIG. 10a and 10b respectively show cell density data for the cast film samples crosslinked either with 0.5% (w/w) or 2.5% (w/w) Genipin at 4, 24 and 48 h, as compared to a Sacran control layer. According to the results from dissolution tests, it is reasonable to assume that samples swell, partially dissolve and mainly release Sacran, during cell adhesion and proliferation tests. Swelling and dissolution behavior of samples affect cells adhesion and proliferation. For all samples, cell attachment increases with increasing cell culture time and is lower than for the Sacran control layer. More precisely, the cell density data for the different PDRN/Sacran - 2.5 Genipin blends are similar after 4 h, whereas they show a slight decrease with decreasing PDRN content after 24-48 h. After 24-48 h, cell density on PDRN/Sacran 60/40-2.5 Genipin and PDRN/Sacran 40/60 - 2.5 Genipin samples, as well as that on PDRN/Sacran 40/60 - 2.5 Genipin and PDRN/Sacran 80/20 - 2.5 Genipin samples are not significantly different ($p > 0.05$). On the other hand, the cell density of the PDRN/Sacran 60/40 - 2.5 Genipin blend significantly differs from that of the PDRN/Sacran 20/80 - 2.5 Genipin after 24-48 h. For the PDRN/Sacran - 0.5 Genipin blend substrates, the cell density decreases with increasing Sacran content from 40 wt.% to 60 wt.%, whereas its maximum value is found for the PDRN/Sacran 20/80 - 0.5 Genipin blend. Fibroblasts adhesion and proliferation is affected by surface chemistry of films (which depends on composition and processing) as well as by partial dissolution of poorly crosslinked film samples, which influences surface composition, and interferes with cell attachment through Sacran releases.

The crosslinked samples were found to be biocompatible and support cell adhesion and proliferation particularly well. As such, embodiments of the present invention are considered to be particularly suitable for use as scaffolds in tissue engineering.

BLANK GENIPIN-CROSSLINKED PDRN/SACRAN AND EGF-LOADED GENIPIN-CROSSLINKED PDRN/SACRAN HYDROGEL IMPLANT AND THEIR IN-VIVO EFFECTS ON ANIMAL WOUND MODEL

In order to investigate the in-vivo wound healing effects of embodiments of the present invention, two hydrogel implant versions were developed: 7 being blank Genipin-crosslinked PDRN/Sacran hydrogel implants, the other 7 being EGF-loaded Genipin-crosslinked PDRN/Sacran hydrogel implants.

The blank hydrogel implants were constructed as aforementioned, with dimensions (w) 25mm x (d) 25mm x (h) 1mm and a PDRN/Sacran 40/60 content. The EGF-loaded hydrogel implants were also constructed as aforementioned with the addition of loading EGF at 0.001% w/w concentration by dialyzing EGF (Perpetuum) into the PDRN/Sacran solution, following by loading into Genipin Solution, followed by the gelling phase. The EGF-loaded PDRN/Sacran hydrogel implants were constructed with same dimensions, being; (w) 25mm x (d) 25mm x (h) 1mm and a PDRN/Sacran 40/60 content.

The wound healing effects of the present invention were assessed in male adult Wistar rats (Germany, 10 weeks old, 280-320g, n = 21). The rats were housed under a 14h light /10h dark cycle with access to water and food "ad libitum". Before the study, all animals were in quarantine for two weeks. Furthermore, all manipulations were performed under sterile conditions in accordance with German guidelines for the care and use of laboratory animals and under protocol approved by the ethical committee of Charité - Universitätsmedizin (Berlin). Transplantation experiments with blank Genipin-crosslinked PDRN/Sacran hydrogel implants, EGF-loaded Genipin-crosslinked PDRN/Sacran hydrogel implants, and control samples were carried out under anesthesia with isoflurane gas (300-400 mL/min, isoflurane: 1.5 -2.0 %) using a Univentor anesthesia unit (Univentor).

For the purpose of the study, standard full-thickness wounds were created. Briefly, on day 0, rats were anesthetized, the dorsum were shaved and cleaned using a saline-drenched cotton gauze and further swabbed with 70% isopropyl alcohol. Single full-thickness wounds with dimensions, being; (w) 25mm x (d) 25mm x (h) 1mm, were created on the left dorsal flank skin of each rat to the depth of the loose subcutaneous tissues, and were left open, see FIG. 11.

The rats were divided into three groups (7 rats per group). The excised full-thickness wounds of the control group were dressed with sterile, medical gauze. The blank group was treated with the Genipin-crosslinked PDRN/Sacran hydrogel implant, while the EGF-loaded group was treated with the EGF-loaded Genipin-crosslinked PDRN/Sacran hydrogel implant. To prevent the rats from removing the treatment materials, a pief of Sorbaview (Centurion) was placed on top of all wounds. All rats were housed in individual cages, maintained at an ambient temperature (22°C), with 14h light / 10h dark cycles and "ad libitum" access to water and food.

For biochemical-, histopathological-, and antioxidant enzyme analysis, 3 rats per group were sacrificed under anesthesia on the 4th and 8th day after surgery. The wound collagen content, granulation tissue formation, wound maturity, and superoxide dismutase and catalase activity were investigated in detail as described hereunder.

For the histopathological alteration evaluation, adjacent skin fragments were removed together with the wound

area. Following standard protocol, the collected samples were fixed in 15% buffered formalin (Sigma-Aldrich), embedded in paraffin (Sigma-Aldrich) and then perpendicularly sectioned to the wound surface into thin sections. All tissues were stained with hematoxylin and eosin (Sigma-Aldrich) and analyzed using a light microscope (Thunder, Leica). To visualize macrophages, the tissue sections were also stained with mouse anti-Iba IgG antibodies (Merck), and Alexa488-conjugated anti-mouse IgG antibodies (Thermo Fisher), followed by counter-staining with Hoechst 3342 fluorescent solution (Thermo Fisher), according to the instructions of the manufacturer.

Immediately after wounding of the rats, subsequently after dressing removal, and cleansing with sterile saline solution (Sigma-Aldrich) on the 4th and 8th day after surgery, the wound areas were photographed with a digital camera (Panasonic HC x1000) accompanied by identity plates and calibration bar. Based on scaled digital images of each wound, using imageJ analysis software, the wound closures were determined. By measuring the open wound area in each digital image, at each point, the wound closures were calculated. By using the following formula, the open wound area was calculated as % of the original area immediately after wounding on day 0:

$$\%\ \text{wound closure} = \frac{(\text{wound area on day 0} - \text{wound area on day x})}{\text{wound area on day 0}} \times 100$$

The effects of the blank- and EGF loaded hydrogel implants on the process of granulation and re-epithalization were studied to evaluate wound closure in more detail. In hematoxylin- and eosin-stained tissue samples, the thickness of granulation tissue and the extent of re-epithalization were evaluated. As shown in FIG. 12a, 12b and 12c, the granulation of the wound areas were significantly enhanced in wounds after 8-d treatments with both blank- and EGF loaded hydrogel implants but no significant improvement in granulation was apparent in the control samples, which exhibited little to almost no granulation. In the blank group, granulation was significantly better than that of the control group, whereas in the EGF-loaded group, granulation and wound closure was almost completely closed. Re-epithelialization was analyzed in all test groups on days 4 and 8. As shown in FIG. 13, the control group did not show a pronounced epithelial regeneration, whereas in both the blank- and EGF-loaded group, enhanced formation of the epithelial lining was very apparent as early as 4 days after surgery. In all of the blank- and EGF-loaded samples, re-epithelialization was improved by day 8. These results were consistent with the analysis of the residual wound areas. As shown in FIG. 14, the wounds treated with both blank PDRN/Sacran hydrogel implants and EGF-loaded PDRN/Sacran hydrogel implants exhibited a well-defined regenerated and differentiated epidermal layer on day 8, with a fairly higher number of cells and a relatively thicker dermis than the control samples who displayed on-going epithelial layer formation with poor granulation and some edema.

Wound healing progression is shown in FIG. 15. Wounds treated with both blank PDRN/Sacran and EGF-loaded PDRN/Sacran hydrogel implants exhibited noticeable uniform dryness, no indication of pathological fluids oozing out, and no signs of infection or inflammation versus the control group. The wound closure was analyzed in each group as a percentage of the reduction in wounded areas on days 4 and 8, see FIG. 16a. The rats that were treated with blank PDRN/Sacran hydrogel implants showed substantial improvement in wound closure (24.28 $\pm$ 2.14% on day 4; 74.32 $\pm$ 3.08% on day 8), compared to the rats in the control group (5.91 $\pm$ 4.23% on day 4; 19.81 $\pm$ 1.19% on day 8). The rats that were treated with EGF-loaded PDRN/Sacran hydrogel implants showed an even more significant improvement in wound closure (54.32 $\pm$ 4.65% on day 4; 88.04 $\pm$ 6.11% on day 8), compared tot he rats in the control group (idem as aforementioned).

By measuring the open wound areas of the rat groups on days 4 and 8, the residual wound surface was determined, see FIG. 16b. The wounds began to close on day 4 and all residual wound surfaces were reduced in all rat groups by the end of day 8. Significant reductions of the residual wound surface were observed in both the blank PDRN/Sacran and EGF-loaded PDRN/Sacran group at the end of day 8, whereas by great contrast, the largest wound residual surface was observed in the control group, indicating a much slower healing rates.

Obviously, a reduction of the residual wound surface is a key parameter in wound healing, as such it indicates a reduction in inflammation and infection.

To evaluate the effects of both blank PDRN/Sacran and EGF-loaded PDRN/Sacran hydrogel implants on tissue inflammation, each group of rats was tested by Hoechst 33528 and anti-Iba1 antibody staining of body tissue samples. Hematoxylin and eosin staining supported the appearance of enhanced wound healing in the groups that were treated with both blank PDRN/Sacran and EGF-loaded PDRN/Sacran hydrogel implants, versus the control group. The inflammatory response at the implantation site was evaluated in order to better understand the healing effect of the implanted materials.

As shown in FIG. 17, a massive inflammatory response was observed in the control group, with an extreme macrophage accumulation at the wound site. After day 8, the accumulation of macrophages was reduced, but remained significantly higher versus the blank PDRN/Sacran and EGF-loaded group. As by contrast, no accumulation of macrophages was apparent on day 4 for both groups, indicating significant wound healing with the cell proliferation phase

already induced. For both groups, on day 4 a clear granulation effect was noticeable, indicative of the accumulation of anti-inflammatory cells, which by day 8 turned into a uniform defined epithelium, thus sufficiently supporting the improved healing efficacy of both blank PDRN/Sacran and EGF-loaded PDRN/Sacran hydrogel implants.

## Claims

1. A biopolymer comprising:

   a polydeoxyribonucleotide, PDRN, or a derivative or modification thereof, having a molecular weight in the range of 400kDa to 3200 kDa,
   an anionic and ampholytic heteropolysaccharide having sulfate and carboxyl groups and a molecular weight in the range of 14 MDa and 16 MDa,
   wherein said polydeoxyribonucleotide and said heteropolysaccharide are crosslinked by genipin or an analogue or derivative thereof.

2. The biopolymer of claim 1, wherein said heteropolysaccharide is sacran, preferably obtained from Aphanothece Sacrum.

3. The biopolymer of any of the previous claims, wherein said water soluble aglycon is genipin.

4. The biopolymer of any of the previous claims, wherein said PDRN is derived from the testes or milt of Onchorhynchus species.

5. A method of producing a biopolymer, comprising:

   providing a polydeoxyribonucleotide, PDRN, or a derivative or modification thereof, said PDRN having a molecular weight in the range of 400 kDa to 3200 kDa,
   providing an anionic and ampholytic heteropolysaccharide having sulfate and carboxyl groups and a molecular weight in the range of 14MDa and 16MDa, and
   crosslinking said polydeoxyribonucleotide and said heteropolysaccharide by genipin or an analogue or derivative thereof.

6. The method of claim 5, comprising:

   i) dissolving PDRN matrix in deionized water to obtain a first solution of PDRN in the range of 0.005 wt% to 10wt%,
   ii) dissolving Sacran matrix in deionized water to obtain a second solution of Sacran in the range of 0.005 wt% to 10 wt%,
   iii) adding Genipin into a blended solution of said first solution and said second solution to obtain a solution of Genipin in the range of 0.05 wt% to 2.5 wt%, preferably 2.5 wt%,
   iv) crosslinking the solution at a temperature in the range of 30°C to 45°C for at least 12 hours,
   v) gelling the crosslinked solution to obtain a gel comprising biopolymer scaffolds, and
   vi) lyophilizing the scaffolds.

7. The method of claim 6, wherein said first solution and said second solution are blended under continuous stirring at a temperature in the range of 40°C to 50°C, e.g. 45°C, for at least 4 hours, e.g. 8h.

8. The method of claim 6 or claim 7, wherein said solution is crosslinked at a temperature of 37°C for 24 hours.

9. The method of any of the claims 6 to 8, wherein said crosslinked solution is gelled at 4°C for 24 hours, and/or wherein said lyophilizing comprises freezing the gel at a temperature of -80°C for 24 hours and lyophilizing the frozen scaffolds for 48 hours.

10. The method of any of the claims 6 to 9, wherein said solution comprises a PDRN to Sacran ratio in the range of 70 : 30 to 30 : 70, preferably 60 : 40.

11. A composition comprising the biopolymer of any of the claims 1 to 4 for use in a biomedical, cosmetic, food or agricultural product.

**12.** The composition of claim 11, wherein said biopolymer is used as a carrier for direct or sustained release of an active pharmaceutical ingredient, such as an epidermal growth factor.

**13.** A pharmaceutical comprising the biopolymer of any of the claims 1 to 4 for treating inflammation and/or impaired wound healing.

**14.** Use of a composition in accordance with any of the claims 11 to 12 or a pharmaceutical in accordance with claim 13 for the treatment of inflammation and/or impaired wound healing.

**15.** The use of claim 14 of the composition in accordance with any of the claims 11 to 12 or a pharmaceutical in accordance with claim 13 for the treatment of a skin inflammatory disease.

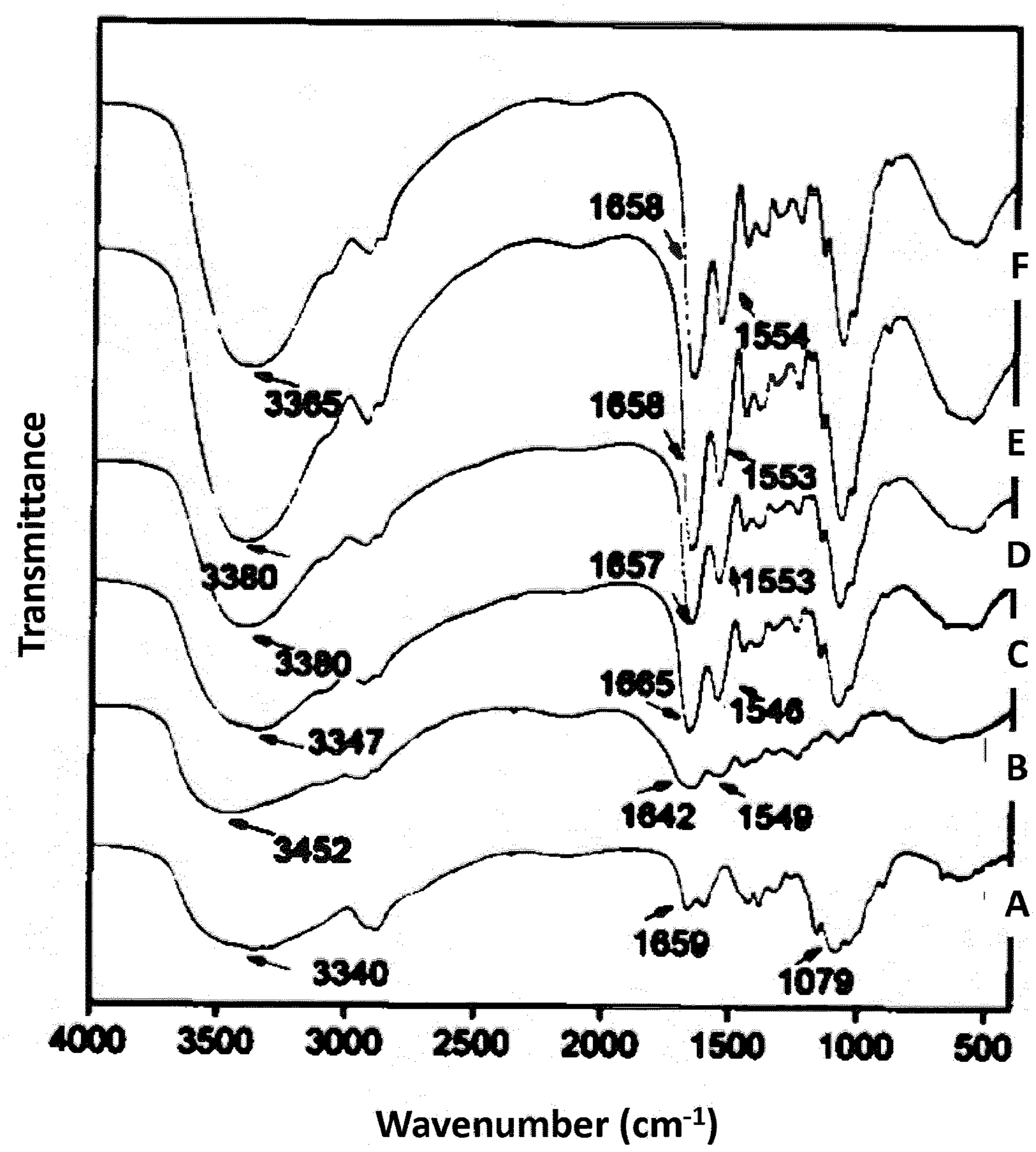
Transmittance
Wavenumber (cm-1)
4000
3500
3000
2500
2000
1500
1000
500
3365
1658
1554
3380
1658
1553
3380
1657
1553
3347
1665
1546
3452
1642
1549
3340
1659
1079
F
E
D
C
B
A

FIG. 1

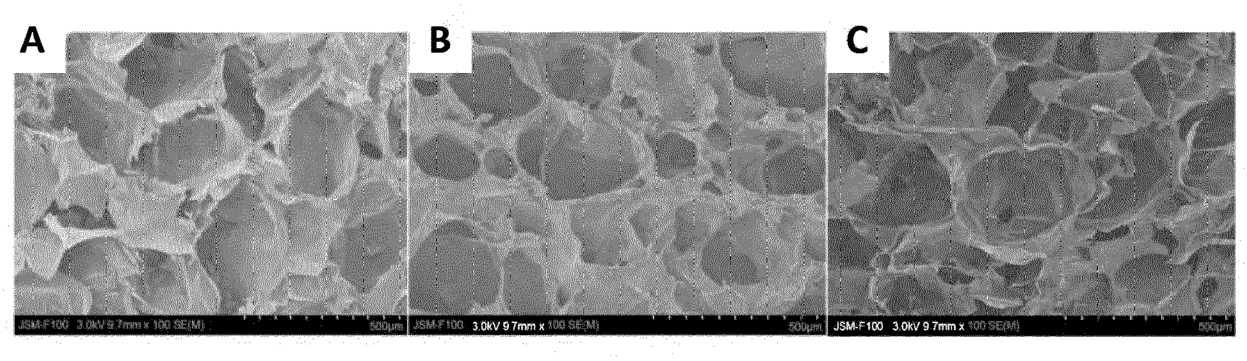
A
B
C

FIG. 2

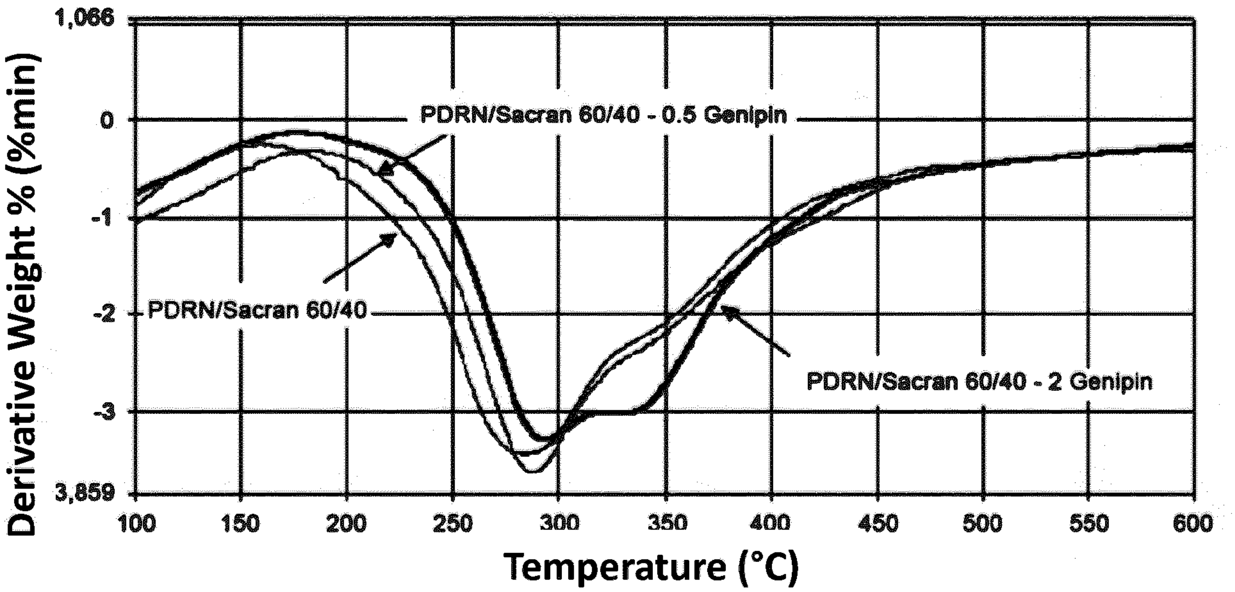


FIG. 3

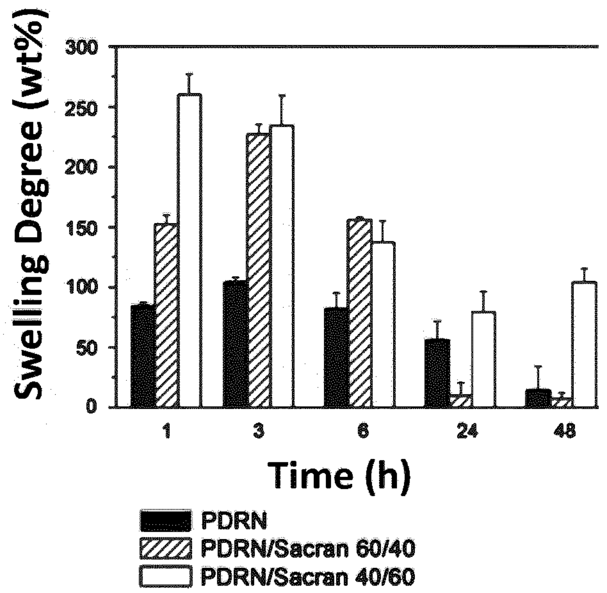


FIG. 4

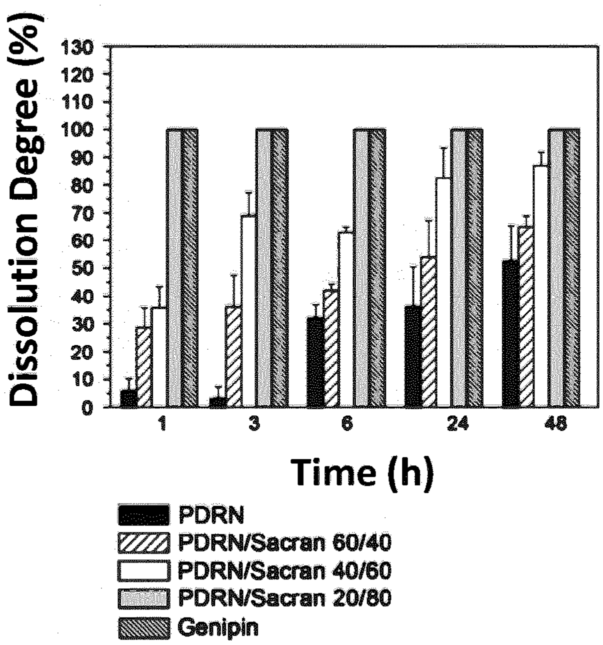


FIG. 5

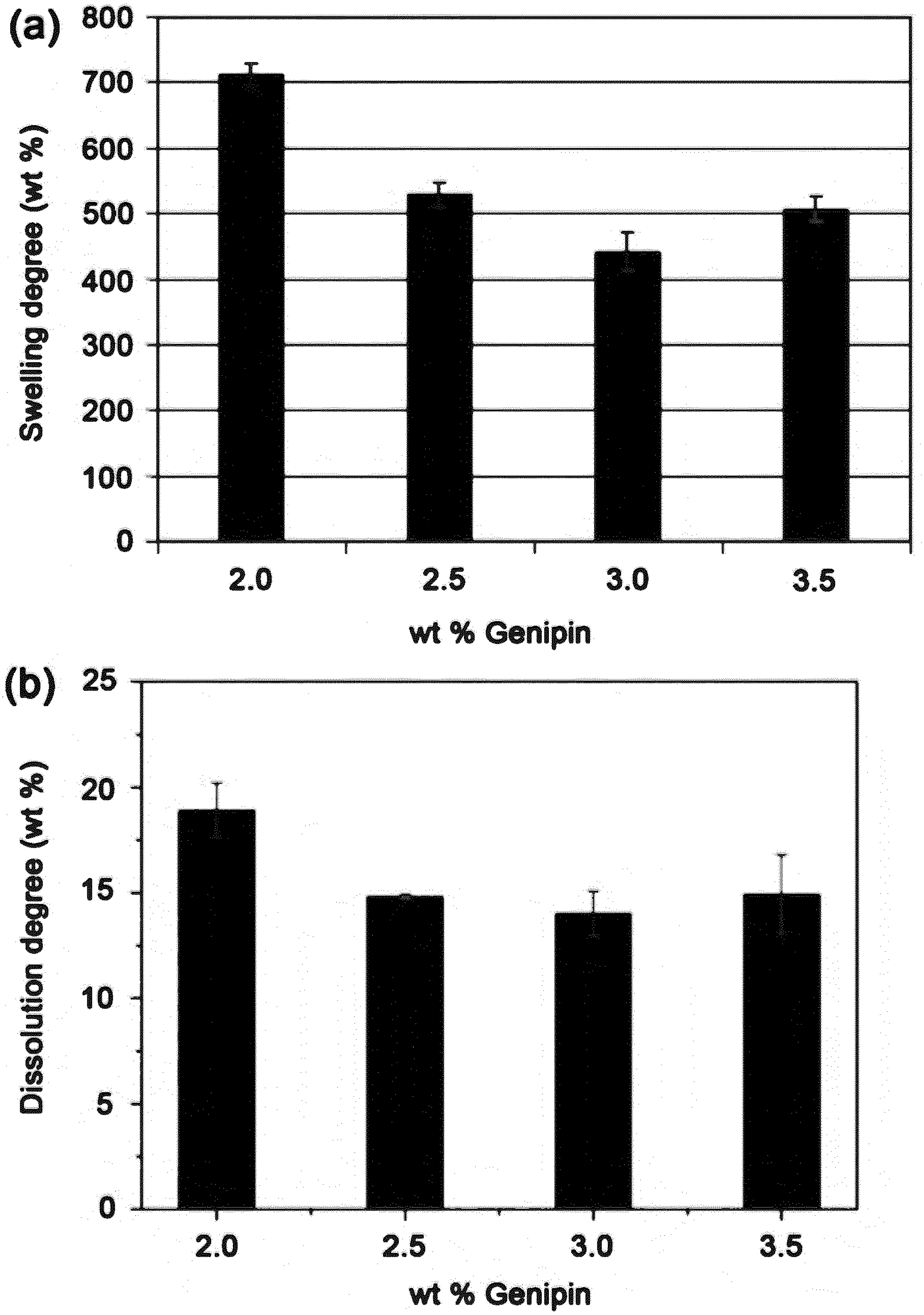
(a)
Swelling degree (wt %)
800
700
600
500
400
300
200
100
0
2.0
2.5
3.0
3.5
wt % Genipin
(b)
Dissolution degree (wt %)
25
20
15
10
5
0
2.0
2.5
3.0
3.5
wt % Genipin

FIG. 6

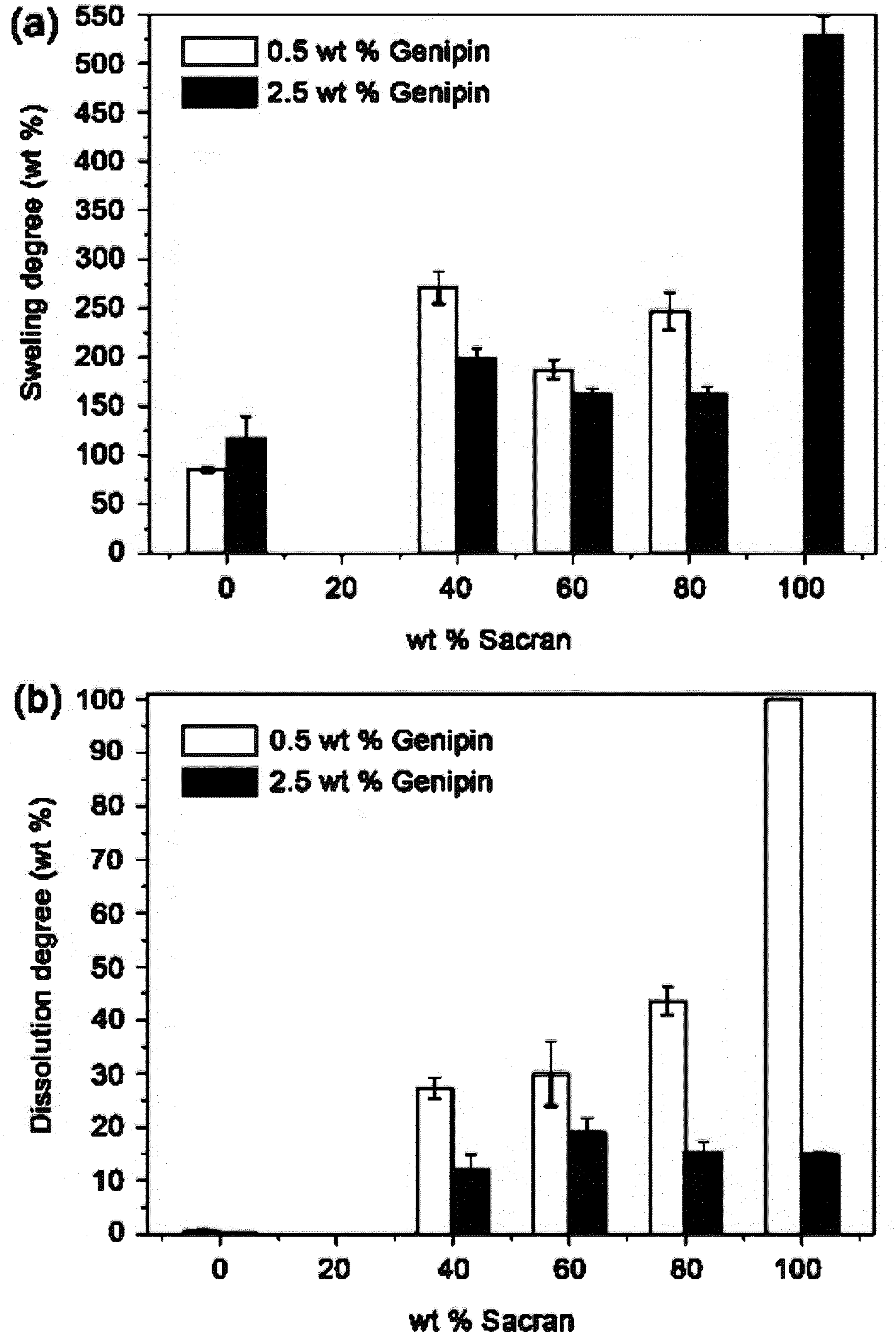
(a)
0.5 wt % Genipin
2.5 wt % Genipin
Sweling degree (wt %)
550
500
450
400
350
300
250
200
150
100
50
0
0
20
40
60
80
100
wt % Sacran
(b)
0.5 wt % Genipin
2.5 wt % Genipin
Dissolution degree (wt %)
100
90
80
70
60
50
40
30
20
10
0
0
20
40
60
80
100
wt % Sacran

FIG. 7

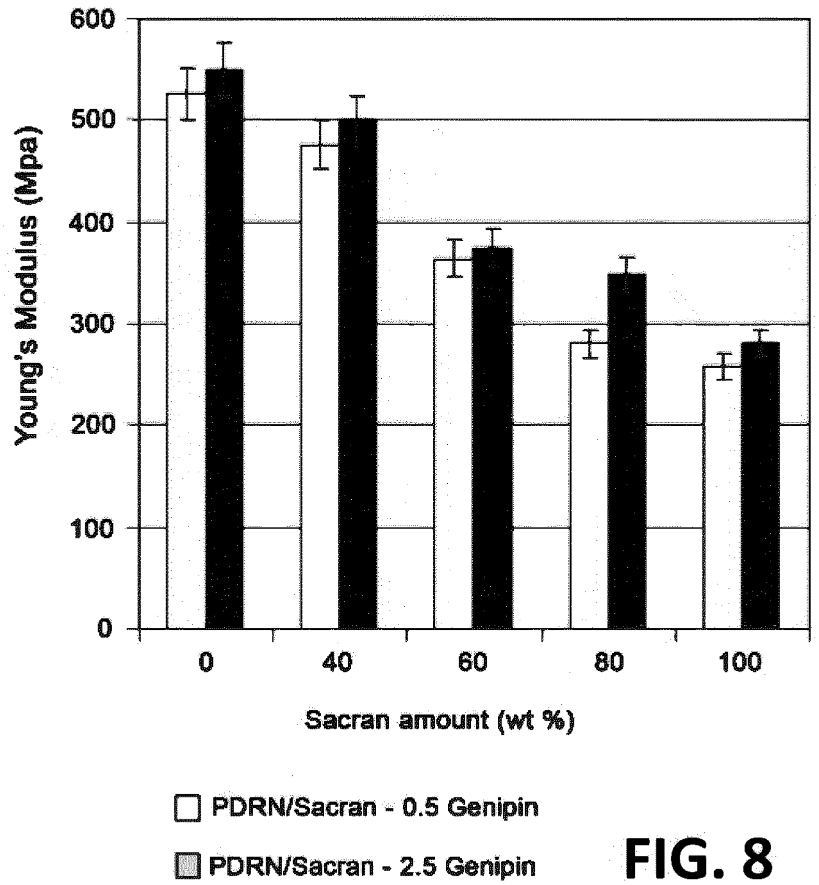

600
500
400
300
200
100
0
Young's Modulus (Mpa)
0
40
60
80
100
Sacran amount (wt %)
PDRN/Sacran - 0.5 Genipin
PDRN/Sacran - 2.5 Genipin


FIG. 8

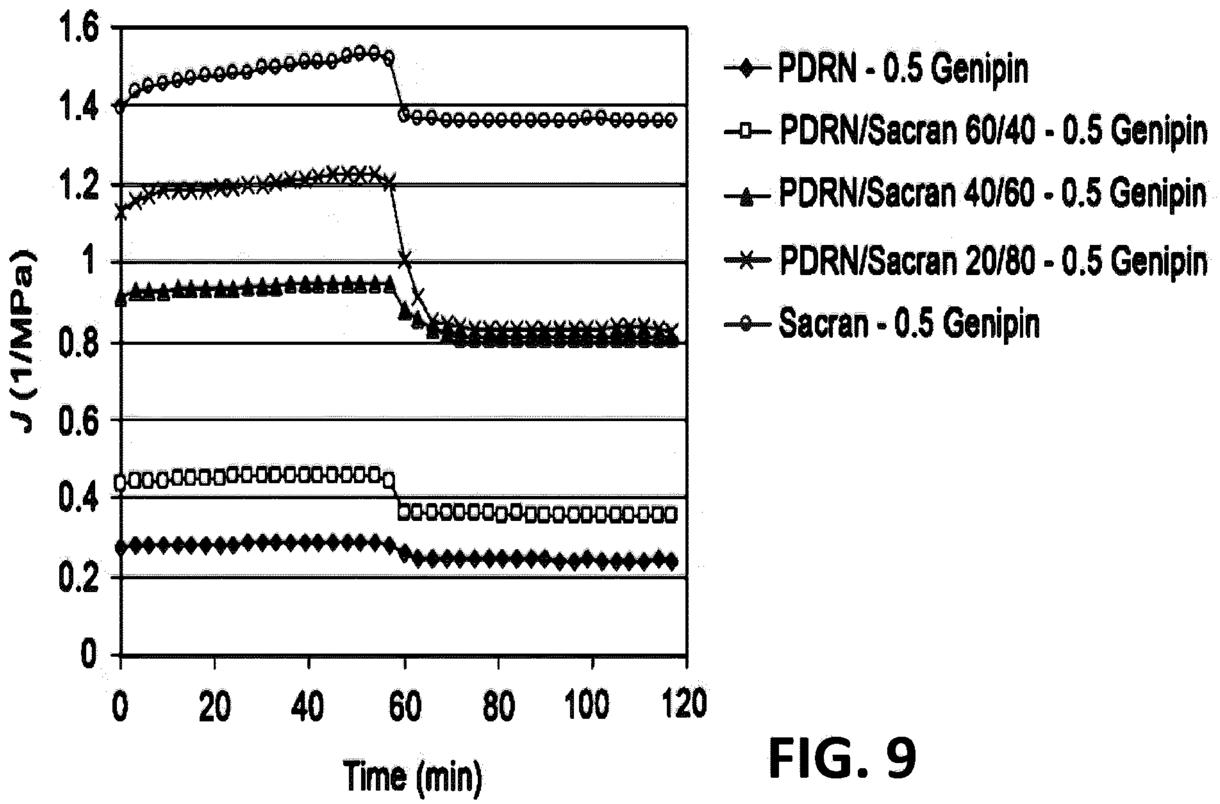

1.6
1.4
1.2
1
0.8
0.6
0.4
0.2
0
J (1/MPa)
0
20
40
60
80
100
120
Time (min)
PDRN - 0.5 Genipin
PDRN/Sacran 60/40 - 0.5 Genipin
PDRN/Sacran 40/60 - 0.5 Genipin
PDRN/Sacran 20/80 - 0.5 Genipin
Sacran - 0.5 Genipin


FIG. 9

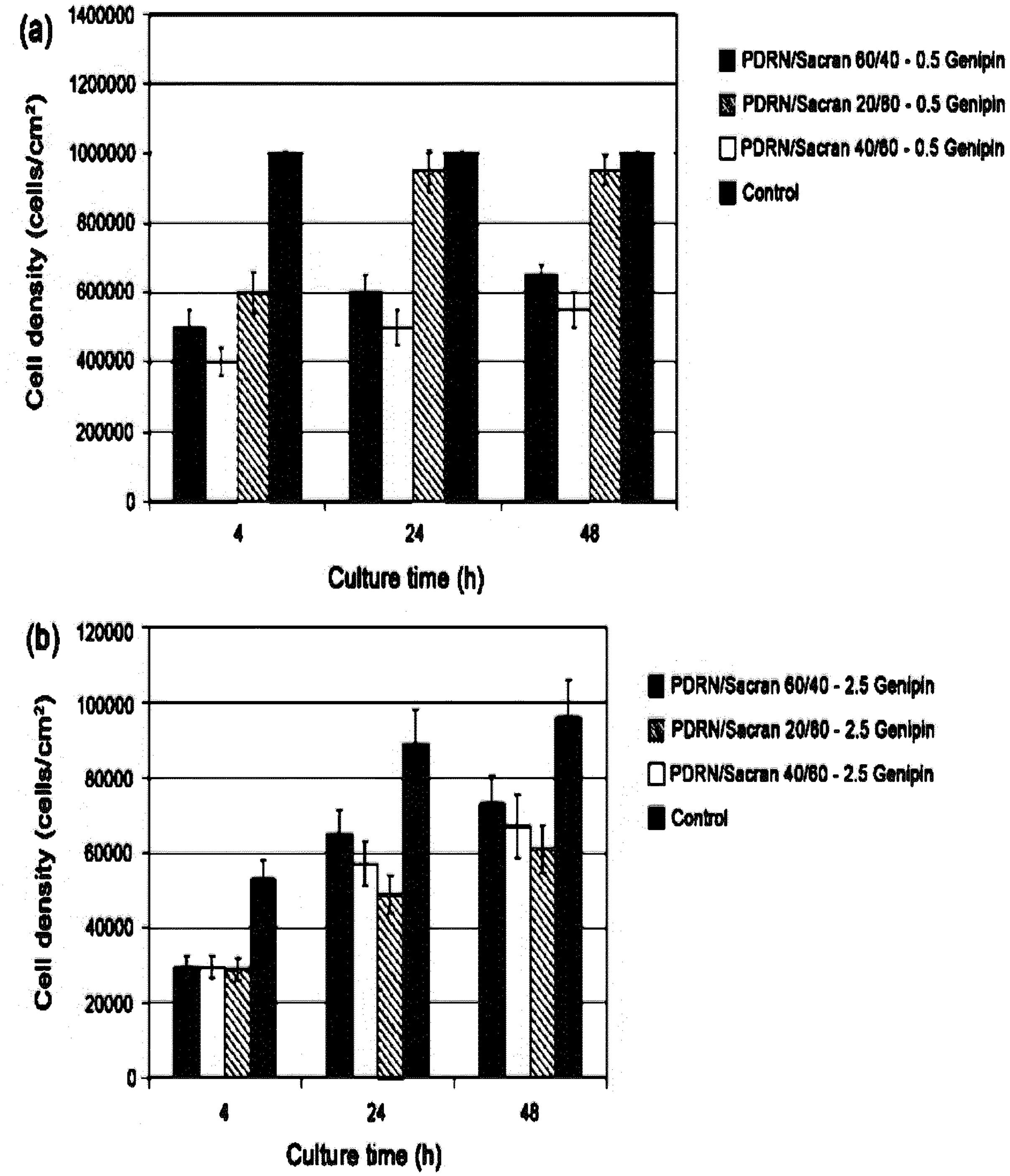
(a)
Cell density (cells/cm²)
1400000
1200000
1000000
800000
600000
400000
200000
0
4
24
48
Culture time (h)
PDRN/Sacran 60/40 - 0.5 Genipin
PDRN/Sacran 20/80 - 0.5 Genipin
PDRN/Sacran 40/60 - 0.5 Genipin
Control
(b)
Cell density (cells/cm²)
120000
100000
80000
60000
40000
20000
0
4
24
48
Culture time (h)
PDRN/Sacran 60/40 - 2.5 Genipin
PDRN/Sacran 20/80 - 2.5 Genipin
PDRN/Sacran 40/60 - 2.5 Genipin
Control

FIG. 10

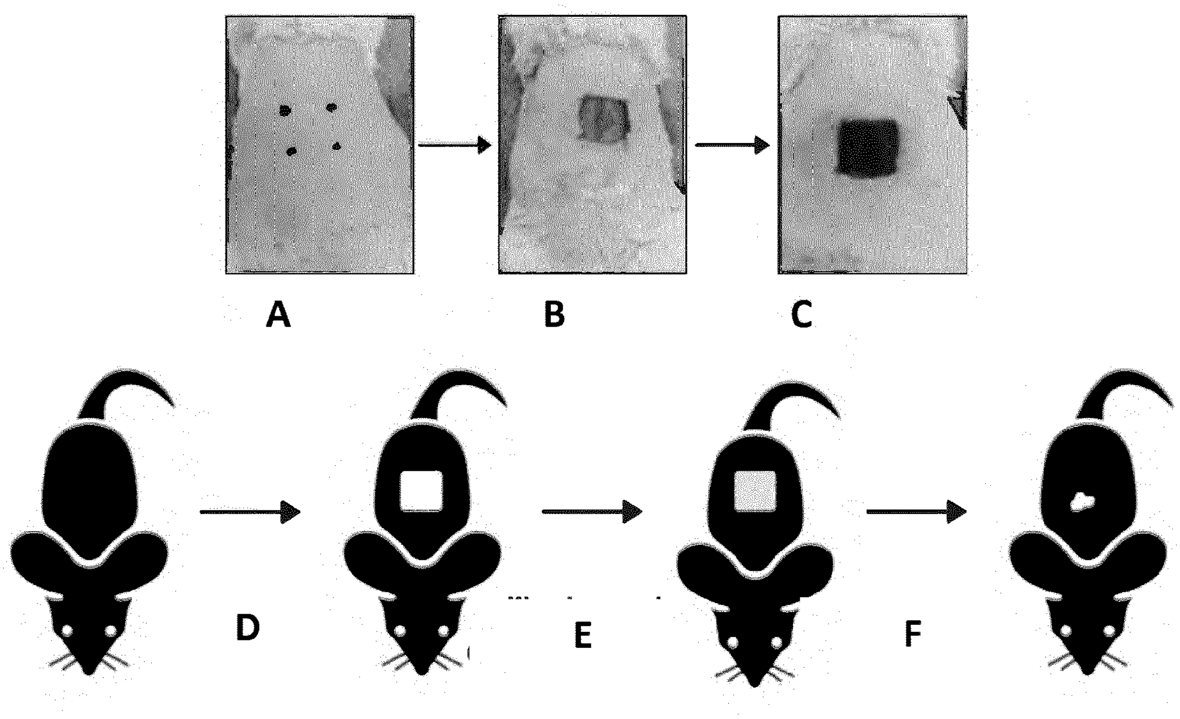
A
B
C
D
E
F

FIG. 11

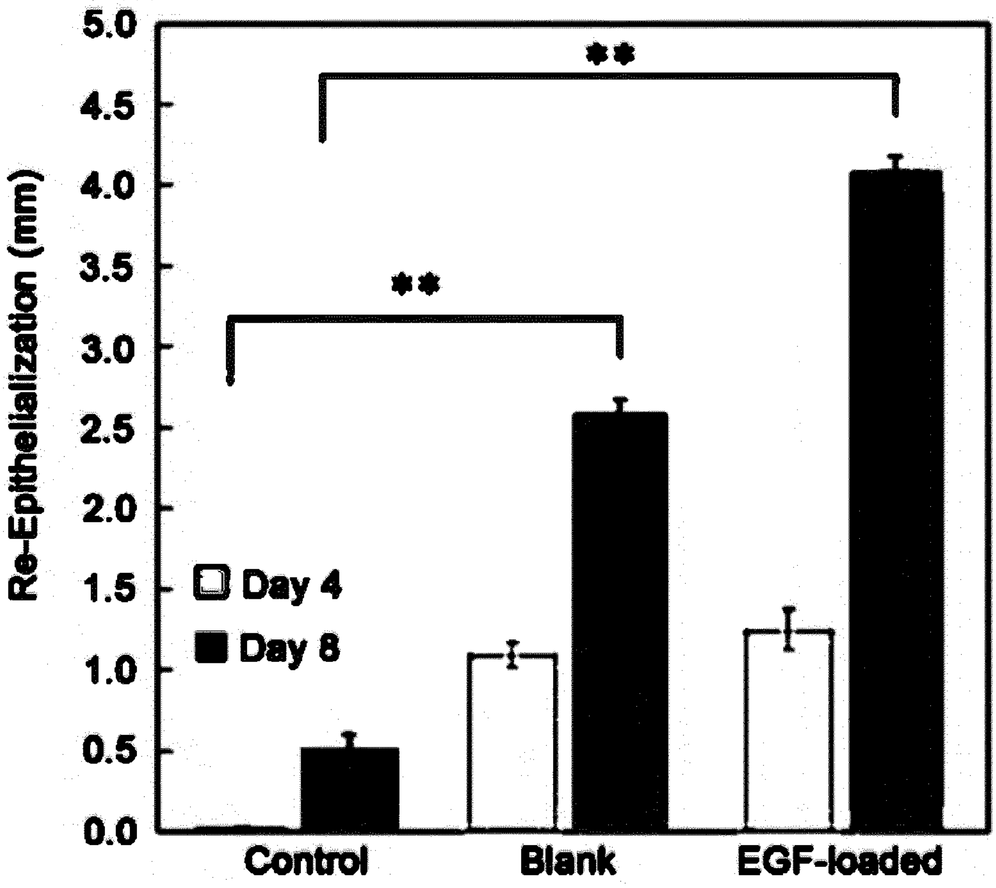
5.0
4.5
4.0
3.5
3.0
2.5
2.0
1.5
1.0
0.5
0.0
Re-Epithelialization (mm)
**
**
Day 4
Day 8
Control
Blank
EGF-loaded

FIG. 13

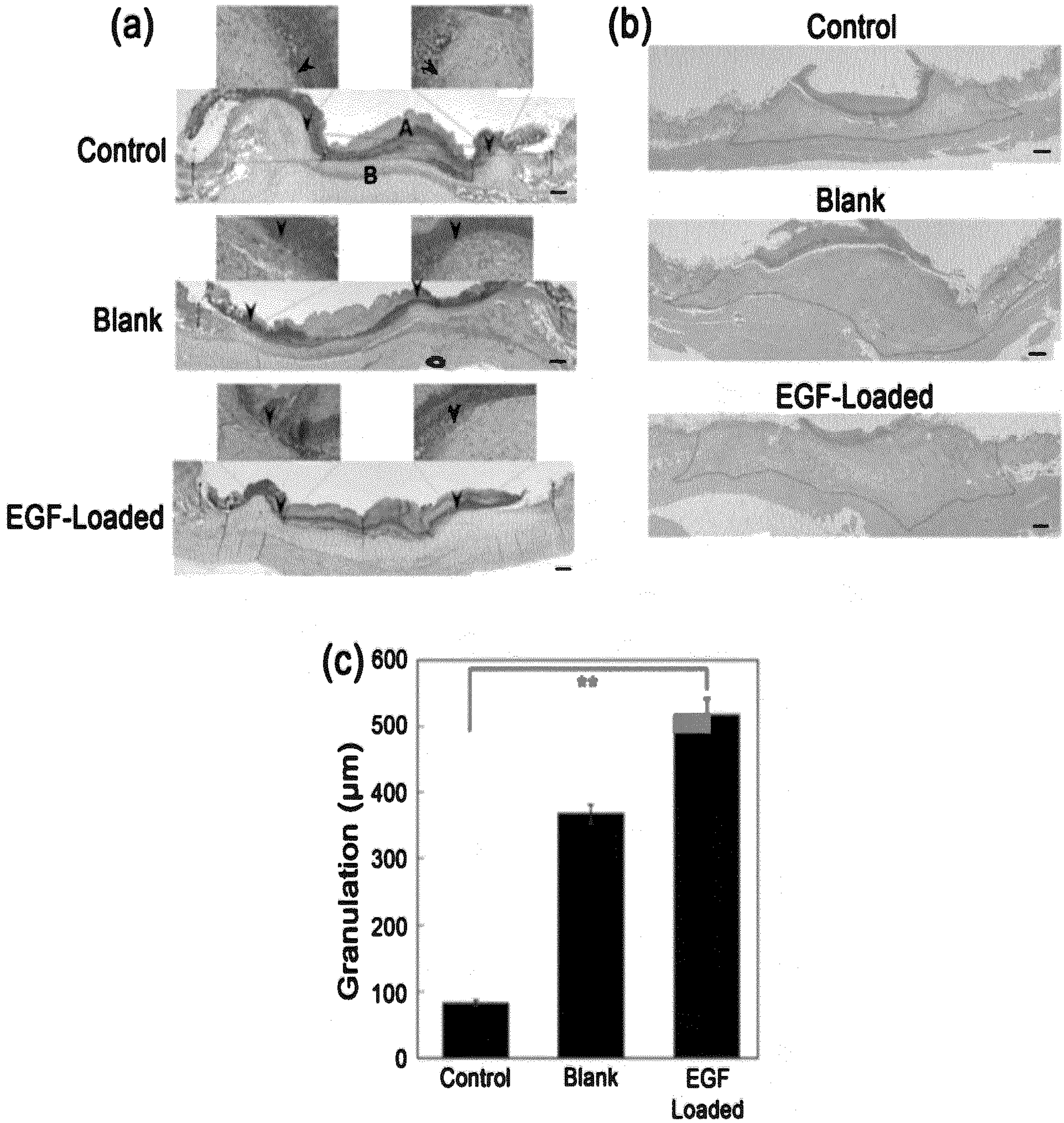
(a)
Control
A
B
Blank
EGF-Loaded
(b)
Control
Blank
EGF-Loaded
(c)
Granulation (μm)
600
500
400
300
200
100
0
**
Control
Blank
EGF
Loaded

FIG. 12

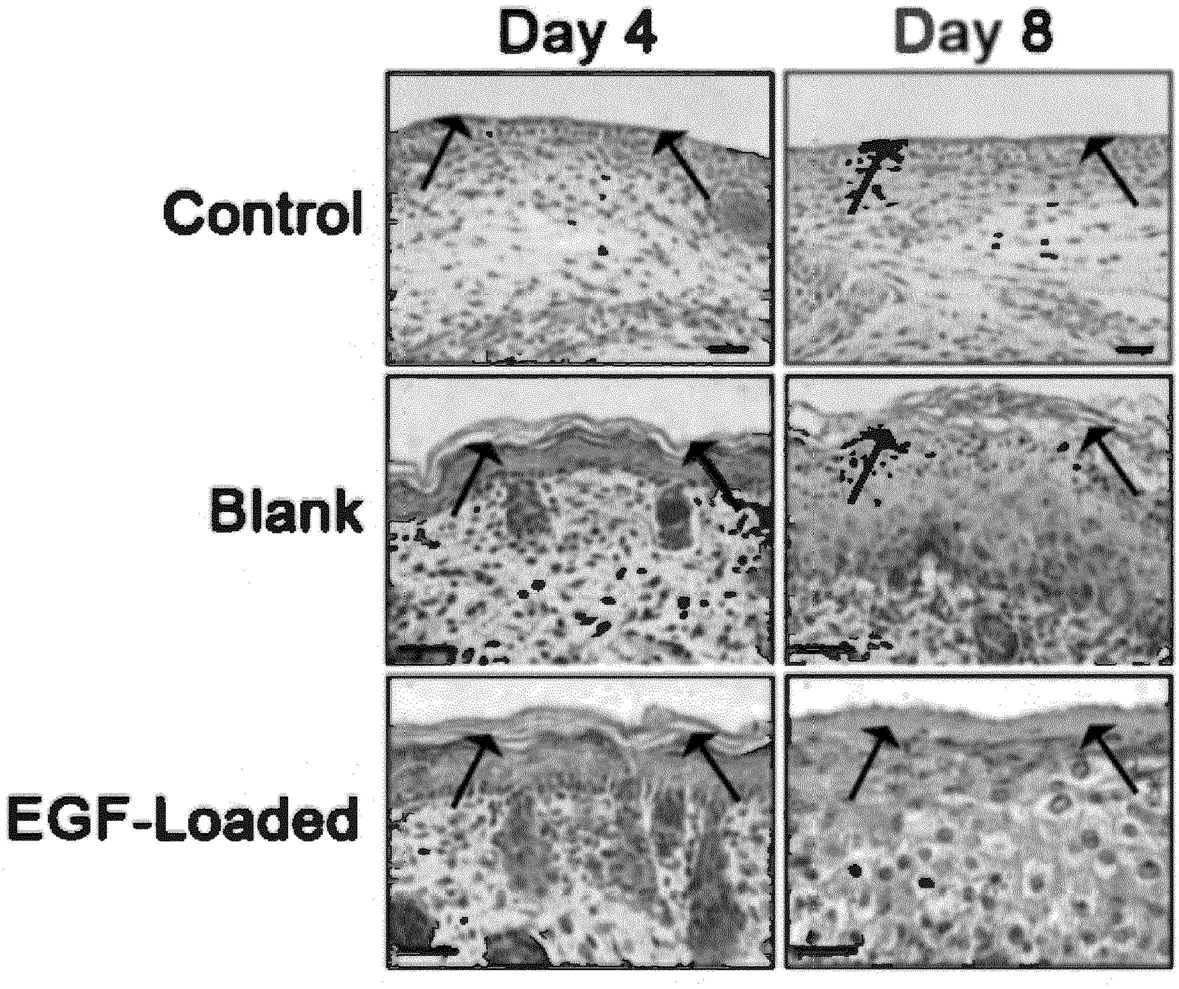


FIG. 14

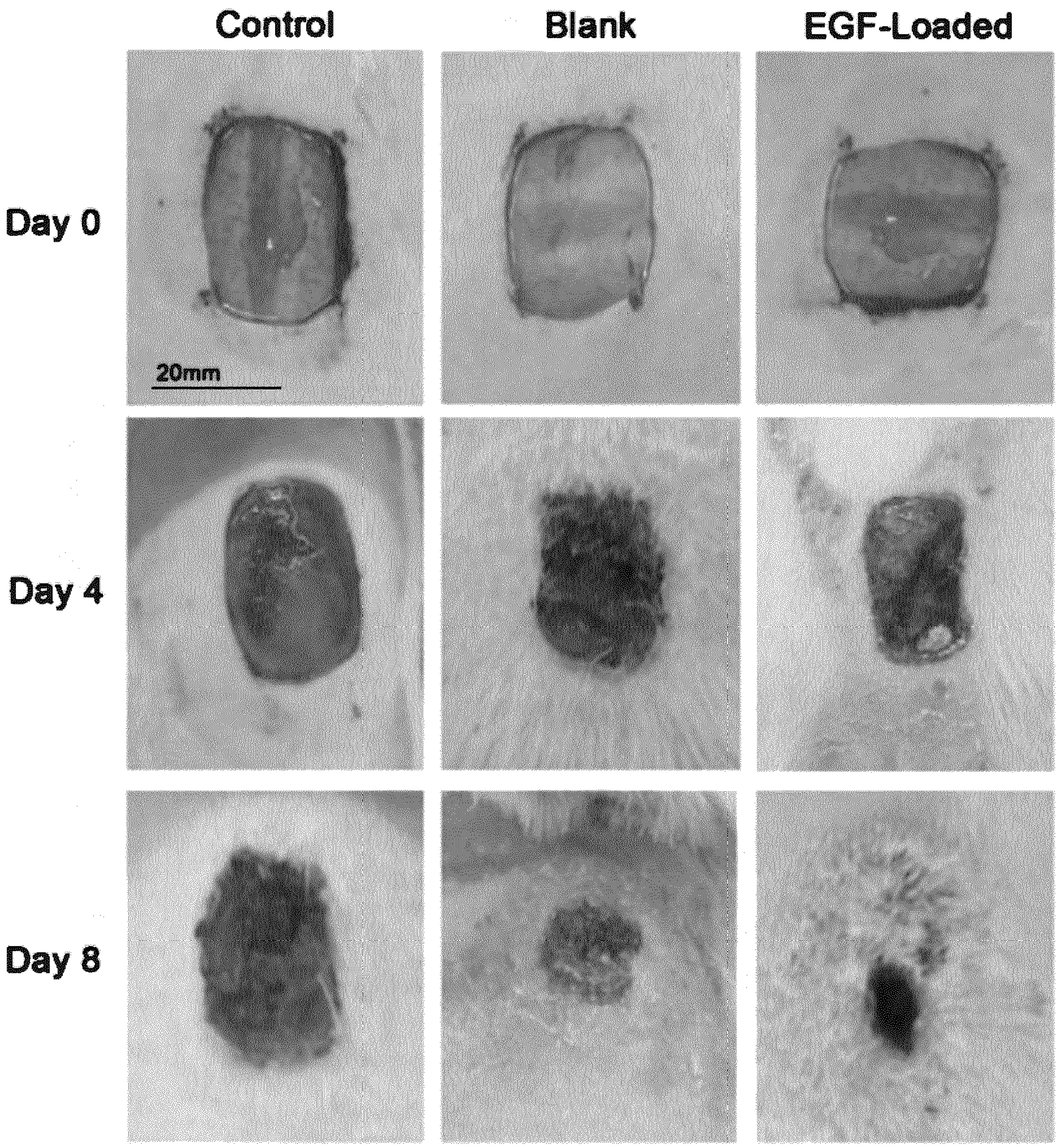


FIG. 15

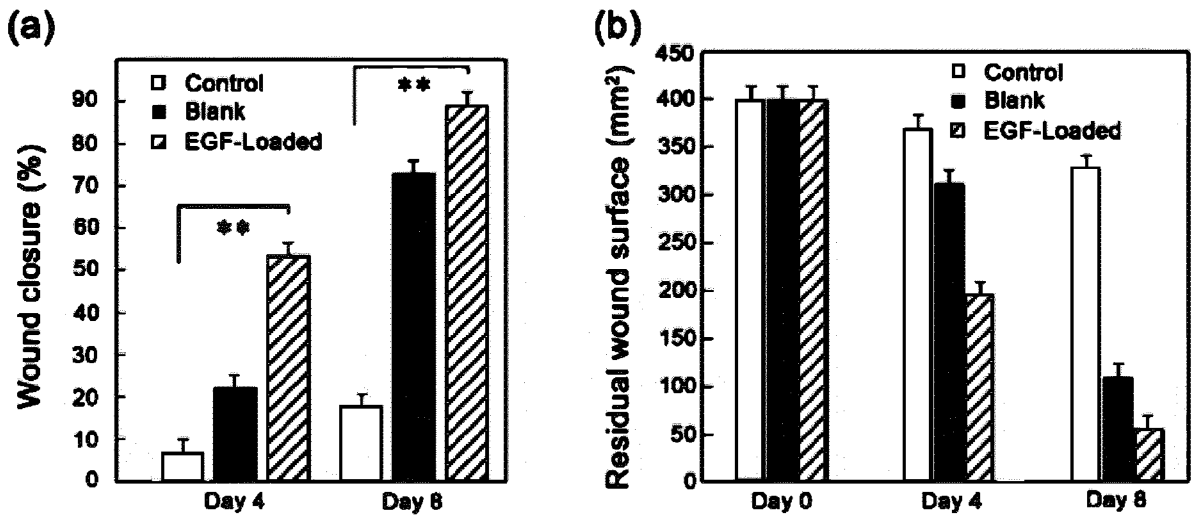
(a)
Control
Blank
EGF-Loaded
**
**
Wound closure (%)
0
10
20
30
40
50
60
70
80
90
Day 4
Day 8
(b)
Control
Blank
EGF-Loaded
Residual wound surface (mm²)
0
50
100
150
200
250
300
350
400
450
Day 0
Day 4
Day 8

FIG. 16

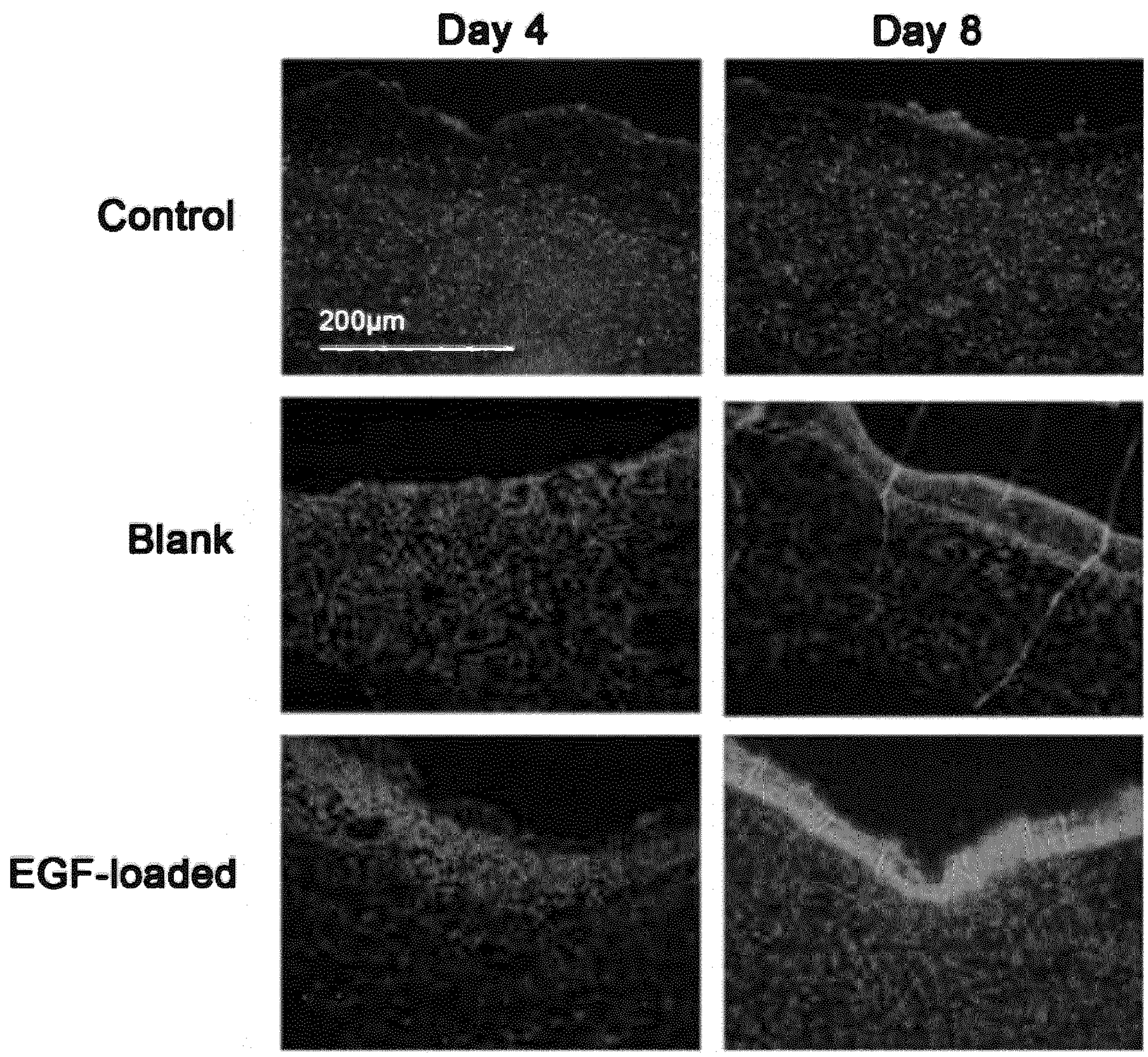
Day 4
Day 8
Control
200μm
Blank
EGF-loaded

FIG. 17

Europäisches Patentamt
European Patent Office
Office européen des brevets

# EUROPEAN SEARCH REPORT

Application Number
EP 20 16 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | EP 3 381 438 A1 (BMI KOREA CO LTD [KR]) 3 October 2018 (2018-10-03)<br>* paragraphs [0001], [0008], [0010] *<br>* pages 2-5; examples 2-5 *<br>* Experimental Example 2; paragraphs [0039] - [0042] *<br>----- | 1-5, 11-15<br>6-10 | INV.<br>C08B37/00<br>A61K8/73<br>A61K31/711<br>A61K47/36<br>A61L27/20<br>C08H99/00<br>C08J3/24<br>C08L5/00<br>C08L99/00 |
| X<br><br>A | US 2005/163818 A1 (SUNG HSING-WEN [TW] ET AL) 28 July 2005 (2005-07-28)<br>* paragraphs [0002] - [0004], [0020] - [0023], [0044] - [0046], [0146] *<br>* pages 1-2 *<br>* paragraphs [0160] - [0162] *<br>----- | 1-5, 11-15<br>6-10 | |
| A | VALDATTA L ET AL: "Evaluation of the efficacy of polydeoxyribonucleotides in the healing process of autologous skin graft donor sites: A pilot study", CURRENT MEDICAL RESEARCH AND OPINION, INFORMA HEALTHCARE, GB, vol. 20, no. 3, 1 March 2004 (2004-03-01), pages 403-408, XP008122537, ISSN: 0300-7995<br>* abstract *<br>* Patients and Methods; page 404 *<br>* Right column, second full paragraph; page 407 *<br>----- | 1-15 | TECHNICAL FIELDS SEARCHED (IPC)<br>C08B<br>C08J<br>A61K<br>A61L<br>A61Q<br>C08H<br>C08L |

The present search report has been drawn up for all claims

1

| Place of search | Date of completion of the search | Examiner |
|---|---|---|
| Munich | 14 September 2020 | Lartigue, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 16 3463

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2020

| Patent document cited in search report | Publication date | Patent family member(s) | Publication date |
|---|---|---|---|
| EP 3381438 A1 | 03-10-2018 | CN 108289825 A | 17-07-2018 |
| | | EP 3381438 A1 | 03-10-2018 |
| | | JP 6660469 B2 | 11-03-2020 |
| | | JP 2019500332 A | 10-01-2019 |
| | | KR 20170060599 A | 01-06-2017 |
| | | KR 20190060752 A | 03-06-2019 |
| | | US 2018325798 A1 | 15-11-2018 |
| | | WO 2017091017 A1 | 01-06-2017 |
| US 2005163818 A1 | 28-07-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7649014 B